# EUROPEAN PATENT SPECIFICATION

(11) **EP 2 968 576 B1**
(45) Date of publication and mention of the grant of the patent: **04.07.2018**
(21) Application number: 14764927.1
(22) Date of filing: 15.03.2014
(51) Int. Cl.: A61K 47/24, A61K 47/08, A61K 39/00, A61K 9/127, A61K 9/51

(54) **SYNTHESIS OF ENT-PROGESTERONE AND INTERMEDIATES THEREOF**
SYNTHESE VON ENT-PROGESTERON UND ZWISCHENPRODUKTE DAVON
SYNTHÈSE D'ENT-PROGESTÉRONE ET D'INTERMÉDIAIRES DE CELLE-CI

(30) Priority: 15.03.2013 US 201361790366 P
(43) Date of publication of application: 20.01.2016
(73) Proprietor: The Florida State University Research Foundation, Incorporated, Tallahassee, FL 32310 (US); Prevacus, Inc., Tallahassee, FL 32308 (US)
(72) Inventor: CRAN, John W., Tallahassee, FL 32301 (US); HAN, Yinglin, Jiugong Beijing 100176 (CN); ZHANG, Faliang, Tongzhou Beijing 101102 (CN)
(74) Representative: Crump, Julian Richard John
(86) International application number: PCT/US2014/030040
(87) International publication number: WO 2014/145302

(56) References cited:
- US-B1- 6 369 247
- US-B1- 6 369 247
- SCOTT D. RYCHNOVSKY, DANIEL E. MICKUS: "Synthesis of ent-cholesterol, the unnatural enantiomer", JOURNAL OF ORGANIC CHEMISTRY, vol. 57, 1 April 1992 (1992-04-01), pages 2732-2736, XP002765366, DOI: 10.1021/jo00035a036
- YANG, Z ET AL.: 'Studies Directed toward Asymmetric Synthesis of Cardioactive Steroids via Anionic Polycyclization' ORGANIC LETTERS vol. 4, no. 26, 2002, pages 4693 - 4696, XP055291777
- MUSUMECI, D ET AL.: 'The oxidation of delta2, delta2,4 and delta4,6 steroids with RuO4' STEROIDS, [Online] vol. 69, 2004, pages 173 - 179 Retrieved from the Internet: <URL:file://gps-fs01/RDSFolderRedirection/s anford.schmolka/Downloads/Ox%20D2,% 20D2,4%20D4,6%20RuO4%20(3).pdf> [retrieved on 2014-06-25]
- HU , Y: 'Studies Toward The Synthesis Of Dumsin. Doctoral Dissertation' 2008, OHIO STATE UNIVERSITY, pages FP - 229, XP055291778 Retrieved from the Internet: <URL:https://etd.ohiolink.edu/rws_etd/docum ent/get/osu1226415494/inline> [retrieved on 2014-06-25]
- AUCHUS, RJ ET AL.: 'The enantiomer of progesterone (ent-progesterone) is a competitive inhibitor of human cytochromes P450c17 and P450c21' ARCHIVES OF BIOCHEMISTRY AND BIOPHYSICS vol. 409, 2003, pages 134 - 144, XP004479092

## Description

### Related Applications

This application claims the benefit of and priority to U.S. Provisional Patent Application No. 61/790,366, filed March 15, 2013, the contents of which are incorporated herein by reference.

### Field of the Invention

The present invention relates to the synthesis of ent-progesterone and intermediates thereof.

### Background

Progesterone is a C-21 steroid hormone involved in the female menstrual cycle, pregnancy and embryogenesis of humans and other species. Progesterone belongs to a class of hormones called progestogens, and is the major naturally occurring human progestogen.

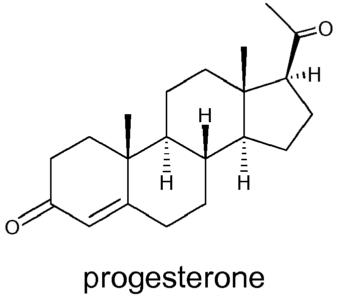

Progesterone is naturally produced by the ovaries of mammals, but may also be produced by some plants and yeast. An economical semi-synthesis of progesterone from the plant steroid diosgenin isolated from yams was developed by Russell Marker in 1940 for the Parke-Davis pharmaceutical company [Marker RE, Krueger J (1940). "Sterols. CXII. Sapogenins. XLI. The Preparation of Trillin and its Conversion to Progesterone". J. Am. Chem. Soc. 62 (12): 3349-3350]. This synthesis is known as the Marker degradation. Additional semi-syntheses of progesterone have also been reported starting from a variety of steroids. For the example, cortisone may be simultaneously deoxygenated at the C-17 and C-21 position by treatment with iodotrimethylsilane in chloroform to produce 11-keto-progesterone (ketogestin), which in turn may be reduced at position-11 to yield progesterone. [Numazawa M, Nagaoka M, Kunitama Y (September 1986). "Regiospecific deoxygenation of the dihydroxyacetone moiety at C-17 of corticoid steroids with iodotrimethylsilane". Chem. Pharm. Bull. 34 (9): 3722-6].

A total synthesis of progesterone was reported in 1971 by W.S. Johnson. [Johnson WS, Gravestock MB, McCarry BE (August 1971). "Acetylenic bond participation in biogenetic-like olefinic cyclizations. II. Synthesis of dl-progesterone". J. Am. Chem. Soc. 93 (17): 4332-4].

The use of progesterone and its analogues have many medical applications, both to address acute situations and to address the long-term decline of natural progesterone levels. Other uses of progesterone include the prevention of preterm birth, to control anovulatury bleeding, to increase skin elasticity and bone strength, and to treat multiple sclerosis.

Progesterone is also useful for the treatment of traumatic brain injury: it reduces poor outcomes following injury by inhibiting inflammatory factors (TNF-α and IL-1β) and subsequently reducing brain edema (Pan, D., et al. (2007), Biomed Environ Sci 20, 432-438; Jiang, C., et al. (2009), Inflamm Res 58, 619-624.) Prog-treated rats have demonstrated significant improvements on a Neurological Severity Score (test for motor and cognitive functioning) following injury (Roof, R. L., et al. (1992), Restor Neurol Neurosci 4, 425-427). Prog effectively attenuates edema in both 25 rodent sexes following injury (Djebaili, M., et al. (2005), J Neurotrauma 22, 106-118). Administering Prog or its derivative allopregnanolone (ALLO) also results in a decreased of the presence of the factors of cell death (caspase-3) and gliosis (GFAP) (Cutler, S. M., et al. (2007), J Neurotrauma 24, 1475-1486) following injury (VanLandingham, J. W., et al. (2007), Neurosci Lett 425, 94-98; Wright, D. W., et al. (2007), Ann Emerg Med 49, 391-402, 402 e391-392). See also, Progesterone for the Treatment of Traumatic Brain Injury (ProTECT III), ClinicalTrials.gov Identifier:NCT00822900; Efficacy and Safety Study of Intravenous Progesterone in Patients With Severe Traumatic Brain Injury (SyNAPSe), ClinicalTrials.gov Identifier:NCT01143064; Progesterone Treatment of Blunt Traumatic Brain Injury, ClinicalTrials.gov Identifier:NCT00048646; and Blood Tests to Study Injury Severity and Outcome in Traumatic Brain Injury Patients (BioProTECT), ClinicalTrials.gov Identifier:NCT01730443. See further, ProTECT™III, Progesterone for the Treatment of Traumatic Brain Injury at http://sitemaker.umich.edu/protect/home; Progesterone for Traumatic Brain Injury Tested in Phase III Clinical Trial at http://www.sciencedaily.com/releases/2010/02/100219204407.htm; BHR Pharma Investigational Traumatic Brain Injury Treatment Receives European Medicines Agency Orphan Medicinal Product Designation at http://finance.yahoo.com/news/bhr-pharma-investigational-traumatic-brain-151600948.html; and BHR Pharma SyNAPSe® Trial DSMB Data Analyses Determine No Safety Issues; Study Should Continue to Conclusion at http://www.prnewswire.com/news-releases/bhr-pharma-synapse-trial-dsmb-data-analyses-determine-no-safety-issues-study-should-continue-to-conclusion-187277871.html.

Progesterone exists in a non-naturally occurring enantiomeric form known as ent-progesterone.

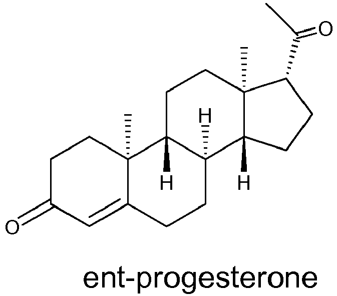

Ent- Progesterone has been shown to have equal efficacy to racemic progesterone in reducing cell death, brain swelling, and inflammation while the enantiomer has three times the antioxidant activity of the racemate. Similarly, ent-progesterone has been found to have fewer sexual side effects such as suppression of spermatogenesis; inhibition of the conversion of testosterone to dihydrotestosterone; reduction in the size of the testes, epididymis, and Leydig cells; and no hyper-coagulative risk as may be seen with racemate progesterone. In addition, utilities for ent-progesterone have been described in U.S. Patent Application No. 13/645,881, which was filed on October 5, 2012 and is entitled "Nasal Delivery Mechanism for Prophylactic and Post-Acute Use for Progesterone and/or Its Enantiomer for Use in Treatment of Mild Traumatic Brain Injuries, U.S. Patent Application No. 13/645,854, which was filed on October 12, 2012 and is entitled "Prophylactic and Post-Acute Use of Progesterone and Its Enantiomer to Better Outcomes Associated with Concussion," and U.S. Patent Application No. 13/645,925, which was filed on October 12, 2012 and is entitled "Prophylactic and Post- 15 Acute Use of Progesterone in Conjunction with Its Enantiomer for Use in Treatment of Traumatic Brain Injuries, the entire contents and disclosures each of which are incorporated herein by reference in their entireties. See also VanLandingham et al., Neuropharmacology, The enantiomer of progesterone acts as a molecular neuroprotectant after traumatic brain injury, 2006, 51, 1078-1085.

Nevertheless, it has been difficult to synthesize ent-progesterone. Previous attempts to synthesize ent-progesterone have suffered from such difficulties as: poor yields, hazardous conditions, hazardous reaction steps, numerous reaction steps and costly reaction steps. These difficulties in synthesizing ent-progesterone have made the commercial use of ent-progesterone and the scale-up of ent-progesterone production unfeasible.

As such, there exists for a need for an efficient synthesis of ent-progesterone.

### Summary of the Invention

In one aspect, the invention provides a method for preparing ent-progesterone comprising the step of reacting a compound of the formula: 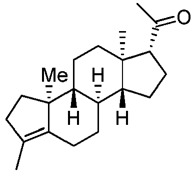 with a ruthenium catalyst and an oxidizing agent to prepare the trienone precursor to ent-progesterone. In certain embodiments, this reaction is performed in the presence of a solvent, including, but not limited to dichoroethane. In other embodiments, the ruthenium catalyst is ruthenium III chloride. In still other embodiments, the oxidizing agent is a metal periodate, including, but not limited to sodium periodate.

In another aspect, the invention provides a method for preparing ent-progesterone comprising the step of reacting a compound of formula A: 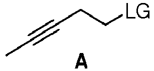 with metal bromide to produce 5-bromopent-2-yne, wherein LG represents a leaving group. In certain embodiments, the leaving group is a tosylate group.

In yet another aspect, the invention provides a method for preparing ent-progesterone comprising the step of reacting a compound of formula D: 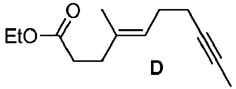 with diisobutylaluminum hydride to form a compound of formula E:

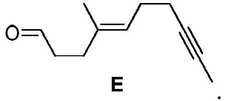

In still yet another aspect, the invention provides a method for preparing ent-progesterone comprising the step of reacting a compound of formula K: 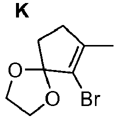 with a compound of formula M: 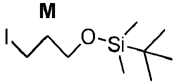 to form a compound of formula N:

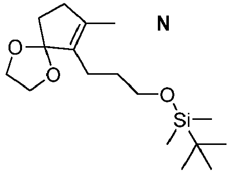

In certain embodiments, the reaction of K and M is in the presence of a lithium compound. In other embodiments, the reaction of K and M is performed in the presence of a solvent. In certain other embodiments the solvent is dimethyl-2-imidazolidinone or 1,3-dimethyl-3,4,5,6-tetrahydro-2(1H)-pyrimidinone or hexamethylphosphoramide or mixtures thereof.

In one embodiment, the invention provides a method for preparing ent-progesterone comprising the step of reacting a compound of the formula: 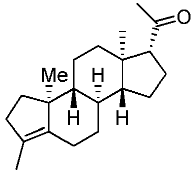 with a ruthenium catalyst and an oxidizing agent to prepare the trienone precursor to ent-progesterone,
and further comprising the step of reacting a compound of formula A: 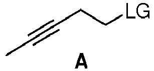 with metal bromide to produce 5-bromopent-2-yne, wherein LG represents a leaving group. In certain embodiments, the leaving group is a tosylate group.

In another embodiment, the invention provides a method for preparing ent-progesterone comprising the step of reacting a compound of the formula: 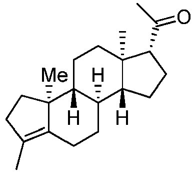 with a ruthenium catalyst and an oxidizing agent to prepare the trienone precursor to ent-progesterone,
and further comprising the step of reacting a compound of formula D: 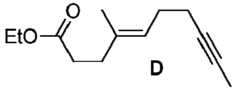 with diisobutylaluminum hydride to form a compound of formula E:

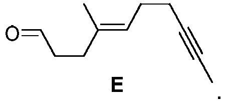

In yet another embodiment, the invention provides a method for preparing ent-progesterone comprising the step of reacting a compound of the formula: 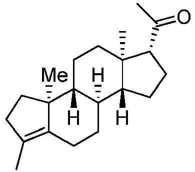 with a ruthenium catalyst and an oxidizing agent to prepare the trienone precursor to ent-progesterone,
and further comprising the step of reacting a compound of formula K: 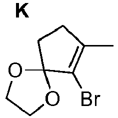 with a compound of formula M: 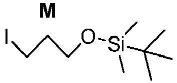 to form a compound of formula N:

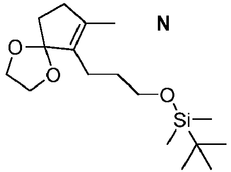

In still yet another embodiment, the invention provides a method for preparing ent-progesterone comprising the step of reacting a compound of the formula: 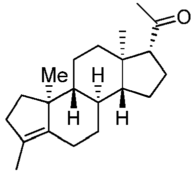 with a ruthenium catalyst and an oxidizing agent to prepare the trienone precursor to ent-progesterone,
and further comprising the step of reacting a compound of formula H: 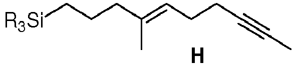 with a compound of formula S : 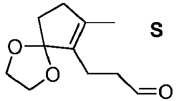 to form a compound of formula T: 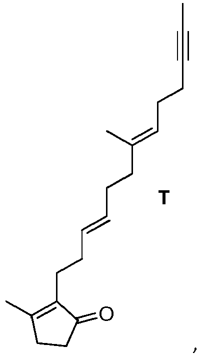 wherein each instance of R is independently a C1-C4 straight or branched alkyl group, or a C3-C8 cycloalkyl group.

In a further embodiment, the invention provides a method for preparing ent-progesterone comprising the step of reacting a compound of the formula: 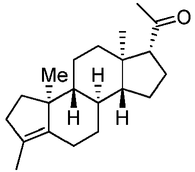 with a ruthenium catalyst and an oxidizing agent to prepare the trienone precursor to ent-progesterone,and further comprising the step of reacting a compound of formula E: 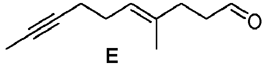 with a compound of formula R : 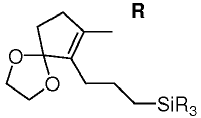 to form a compound of formula T: 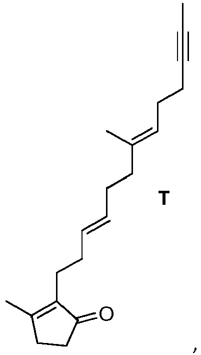 wherein each instance of R is independently a C1-C4 straight or branched alkyl group, or a C3-C8 cycloalkyl group.

In another further embodiment, the invention provides a method for preparing ent-progesterone comprising the step of reacting a compound of the formula: 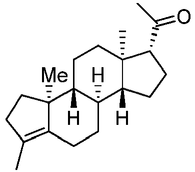 with a ruthenium catalyst and an oxidizing agent to prepare the trienone precursor to ent-progesterone,
and further comprising the step of reacting a compound of formula E: 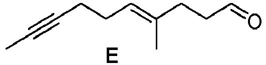 with a compound of formula Q : 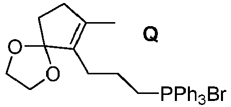 to form a compound of formula T:

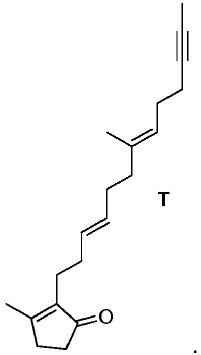

In still yet another embodiment, the invention provides a method for preparing ent-progesterone comprising the step of reacting a compound of the formula: 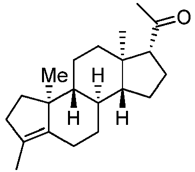 with a ruthenium catalyst and an oxidizing agent to prepare the trienone precursor to ent-progesterone,
and further comprising the step of reacting a compound of formula I: 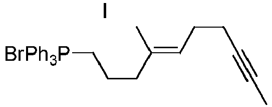 with a compound of formula S: 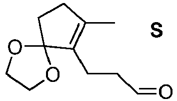 to form a compound of formula T:

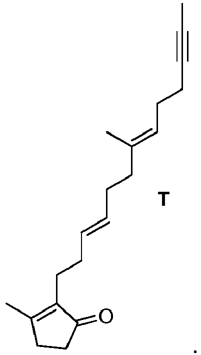

In certain embodiments, the invention provides a method for preparing ent-progesterone comprising two or more of the steps described above. In other embodiments, the invention provides a method for preparing ent-progesterone comprising three or more of the steps described above. In still other embodiments, the invention provides a method for preparing ent-progesterone comprising four or more of the steps described above. In certain embodiments, the invention provides a method for preparing ent-progesterone comprising five of the steps described above.

In accordance with the methods of the invention, enantiomerically-enriched ent-progesterone may be obtained by separation of enatiomers, either of a racemic intermediate or racemic progesterone. Thus, the present invention further contemplates a method of preparing ent-progesterone by isolating ent-progesterone from racemic progesterone. The present invention also contemplates of preparing ent-progesterone by reacting an enantiomerically-enriched intermediate, e.g., intermediate **U** disclosed herein, and transforming the enantiomerically-enriched intermediate through one or more reaction steps to provide ent-progesterone.

In another aspect, the invention provides for one or more intermediates of the synthetic method of the invention. In certain aspects, the intermediate is a compound having one of the following formulas:

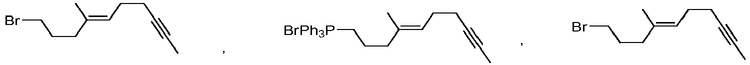

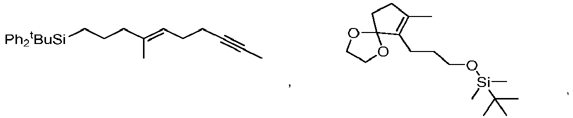

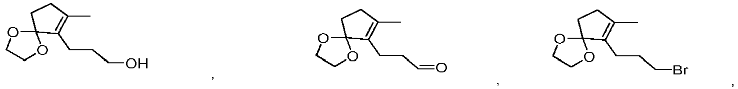

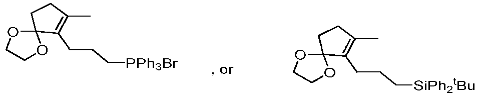

It should be further understood that the above summary of the present invention is not intended to describe each disclosed embodiment or every implementation of the present invention. The description further exemplifies illustrative embodiments. In several places throughout the specification, guidance is provided through examples, which examples may be used in various combinations. In each instance, the examples serve only as representative groups and should not be interpreted as exclusive examples.

### Detailed Description

By way of illustrating and providing a more complete appreciation of the present invention and many of the attendant advantages thereof, the following detailed description and examples are given concerning the novel synthetic synthesis for making ent-progesterone, individual novel steps within the synthetic synthesis and individual novel intermediates formed during the novel synthetic synthesis of the present invention.

As used in the description of the invention and the appended claims, the singular forms "a", "an" and "the" are used interchangeably and intended to include the plural forms as well and fall within each meaning, unless the context clearly indicates otherwise. Also, as used herein, "and/or" refers to and encompasses any and all possible combinations of one or more of the listed items, as well as the lack of combinations when interpreted in the alternative ("or").

As used herein, "at least one" is intended to mean "one or more" of the listed elements.

The term "alkyl" refers to a straight or branched hydrocarbon chain radical consisting solely of carbon and hydrogen atoms, containing no unsaturation, having from one to eight carbon atoms, and which is attached to the rest of the molecule by a single bond, such as illustratively, methyl, ethyl, n-propyl 1-methylethyl (isopropyl), n-butyl, n-pentyl, and 1,1-dimethylethyl (tert-butyl).

The term "cycloalkyl" denotes a non-aromatic mono or multicyclic ring system of 3 to 12 carbon atoms such as cyclopropyl, cyclobutyl, cyclopentyl, cyclohexyl and examples of multicyclic cycloalkyl groups include perhydronapththyl, adamantyl and norbornyl groups bridged cyclic group or spirobicyclic groups e.g. spiro(4,4)non-2-yl.

The term "leaving group," or "LG", as used herein, refers to any group that leaves in the course of a chemical reaction involving the group and includes but is not limited to halogen, brosylate, mesylate, tosylate, triflate, p-nitrobenzoate, phosphonate groups, for example.

Singular word forms are intended to include plural word forms and are likewise used herein interchangeably where appropriate and fall within each meaning, unless expressly stated otherwise.

Except where noted otherwise, capitalized and non-capitalized forms of a term fall within the meaning of the term.

Unless otherwise indicated, it is to be understood that all numbers expressing quantities, ratios, and numerical properties of ingredients, reaction conditions, and so forth used in the specification and claims are contemplated to be able to be modified in all instances by the term "about".

All parts, percentages, ratios, etc. herein are by weight unless indicated otherwise.

### General Preparative Methods

The particular process to be utilized in the preparation of the compounds used in this embodiment of the present invention depends upon the specific compound desired. Such factors as the selection of the specific substituents play a role in the path to be followed in the preparation of the specific compounds of this invention. Those factors are readily recognized by one of ordinary skill in the art.

The compounds of the present invention may be prepared by use of known chemical reactions and procedures. Nevertheless, the following general preparative methods are presented to aid the reader in synthesizing the compounds of the present invention, with more detailed particular examples being presented below in the experimental section describing exemplary working examples.

The compounds of the present invention may be made according to conventional chemical methods, and/or as disclosed below, from starting materials which are either commercially available or producible according to routine, conventional chemical methods. General methods for the preparation of the compounds are given below, and the preparation of representative compounds is specifically illustrated in examples.

Synthetic transformations that may be employed in the synthesis of certain compounds of this invention and in the synthesis of certain intermediates involved in the synthesis of compounds of this invention are known by or accessible to one skilled in the art. Collections of synthetic transformations may be found in compilations, such as:
J. March. Advanced Organic Chemistry, 4th ed.; John Wiley: New York (1992)
R.C. Larock. Comprehensive Organic Transformations, 2nd ed.; Wiley-VCH: New York (1999)
F.A. Carey; R.J. Sundberg. Advanced Organic Chemistry, 2nd ed.; Plenum Press: New York (1984)
T.W. Greene; P.G.M. Wuts. Protective Groups in Organic Synthesis, 3rd ed.; John Wiley: New York (1999)
L.S. Hegedus. Transition Metals in the Synthesis of Complex Organic Molecules, 2nd ed.; University Science Books: Mill Valley, CA (1994)
L.A. Paquette, Ed. The Encyclopedia of Reagents for Organic Synthesis; John Wiley: New York (1994)
A.R. Katritzky; O. Meth-Cohn; C.W. Rees, Eds. Comprehensive Organic Functional Group Transformations; Pergamon Press: Oxford, UK (1995)
G. Wilkinson; F.G A. Stone; E.W. Abel, Eds. Comprehensive Organometallic Chemistry; Pergamon Press: Oxford, UK (1982)
B.M. Trost; I. Fleming. Comprehensive Organic Synthesis; Pergamon Press: Oxford, UK (1991)
A.R. Katritzky; C.W. Rees Eds. Comprehensive Heterocylic Chemistry; Pergamon Press: Oxford, UK (1984)
A.R. Katritzky; C.W. Rees; E.F.V. Scriven, Eds. Comprehensive Heterocylic Chemistry II; Pergamon Press: Oxford, UK (1996)
C. Hansch; P.G. Sammes; J.B. Taylor, Eds. Comprehensive Medicinal Chemistry: Pergamon Press: Oxford, UK (1990), each of which is incorporated herein by reference in its entirety.

In addition, recurring reviews of synthetic methodology and related topics include Organic Reactions; John Wiley: New York; Organic Syntheses; John Wiley: New York; Reagents for Organic Synthesis: John Wiley: New York; The Total Synthesis of Natural Products; John Wiley: New York; The Organic Chemistry of Drug Synthesis; John Wiley: New York; Annual Reports in Organic Synthesis; Academic Press: San Diego CA; and Methoden der Organischen Chemie (Houben-Weyl); Thieme: Stuttgart, Germany. Furthermore, databases of synthetic transformations include Chemical Abstracts, each of which is incorporated herein by reference in its entirety and which may be searched using either CAS OnLine or SciFinder, Handbuch der Organischen Chemie (Beilstein), and which may be searched using SpotFire, and REACCS.

The inventive methods of the present invention to make ent-progesterone are illustrated in Reaction Schemes 1 through 8. The inventive methods include a number of intermediates and reaction methods which enable more efficient and less costly synthesis than heretofore known.

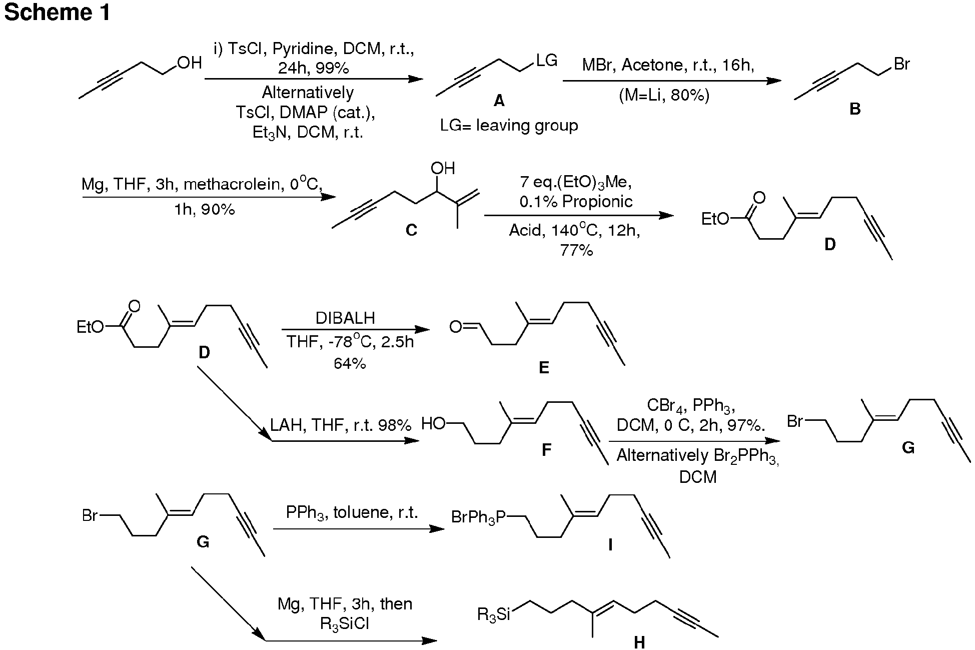

In Scheme 1, 3-pentyn-1-ol is converted to pent-3-ynyl 4-methylbenzenesulfonate (Intermediate A) by tosylation of the hydroxyl group. The tosyl group of Intermediate A is then brominated to form 5-bromopent-2-yne (Intermediate B). Intermediate B is reacted with methacolein to produce 2-methyloct-1-en-6-yn-3-ol (Intermediate C) via a Grignard reaction. Intermediate C is reacted with trimethylorthoacetate to produce (E)-ethyl 4-methyldec-4-en-8-ynoate (Intermediate D).

Next, Intermediate D is reduced to form (E)-4-methyldec-4-en-8-ynal (Intermediate E) or (E)-4-methyldec-4-en-8-yn-1-ol (Intermediate F); either of which may be brominated to form (E)-10-bromo-7-methyldec-6-en-2-yne (Intermediate G).

Finally, Intermediate G is reacted to form an intermediate having a bulky phosphorous or silicon group: Intermediate I or Intermediate H.

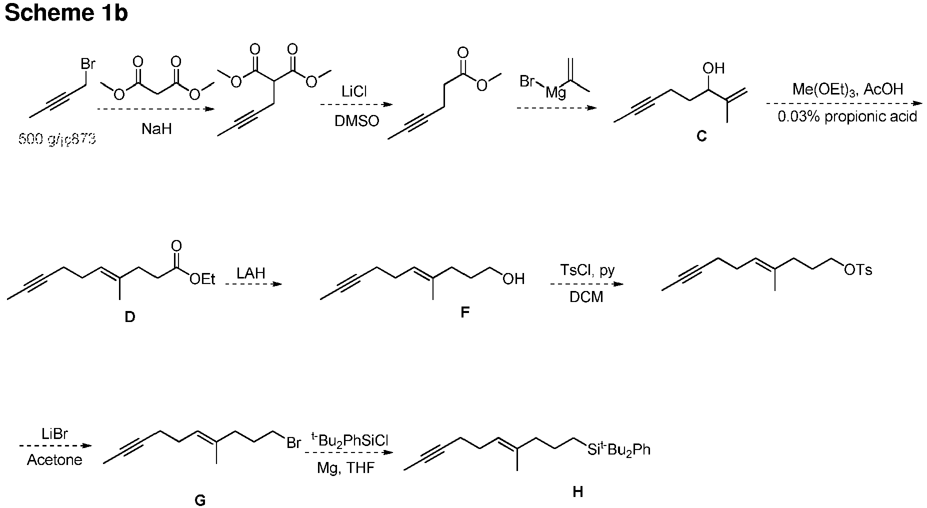

An alternative to Scheme 1 above, Intermediate H may be prepared as show in Scheme 1b. In Scheme 1b, Intermediate C is prepared by reacting 1-bromobut-2-yne with dimethylmalonate in the presence of sodium hydride to produce a substituted malonate which is then reacted with lithium chloride followed by a Grignard reagent. Similarly, Intermediate G is prepared by tosylation followed by bromination of Intermediate F.

In the second phase of the synthesis, shown in Scheme 2 below, methyl cyclopentenone is converted to tert-butyldimethyl(3-(7-methyl-1,4-dioxaspiro[4.4]non-6-en-6-yl)propoxy)silane (intermediate N) via bromination of the double bond, followed by glycolization of the ketone.

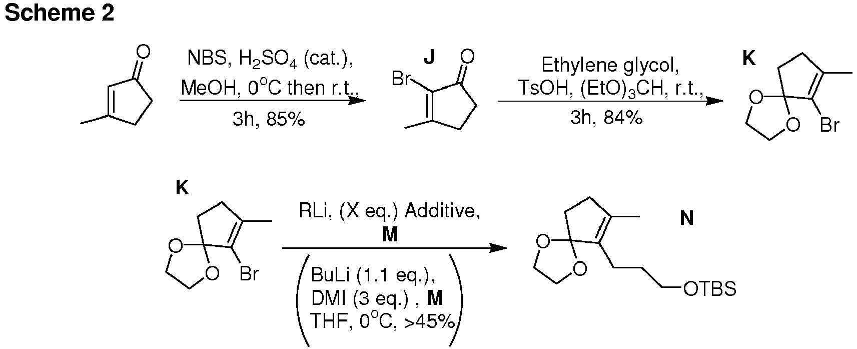

The conversion from 6-bromo-7-methyl-1,4-dioxaspiro[4.4]non-6-ene (Intermediate K) to intermediate N utilizes tert-butyl(3-iodopropoxy)dimethylsilane (Intermediate M) produced as shown in Scheme 3, below.

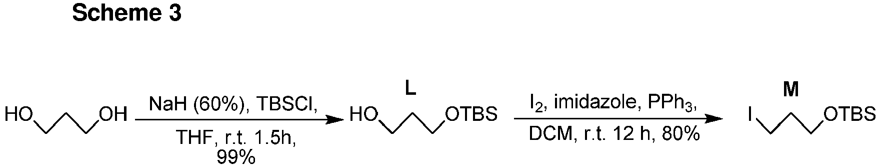

In Scheme 3, 1,3-propanediol is reacted with tert-butyldimethylsilyl chloride followed by reaction with Iodine to produce tert-butyl(3-iodopropoxy)dimethylsilane (Intermediate M).

Compound N may also be prepared using an alternate preparation shown in Scheme 4, below.

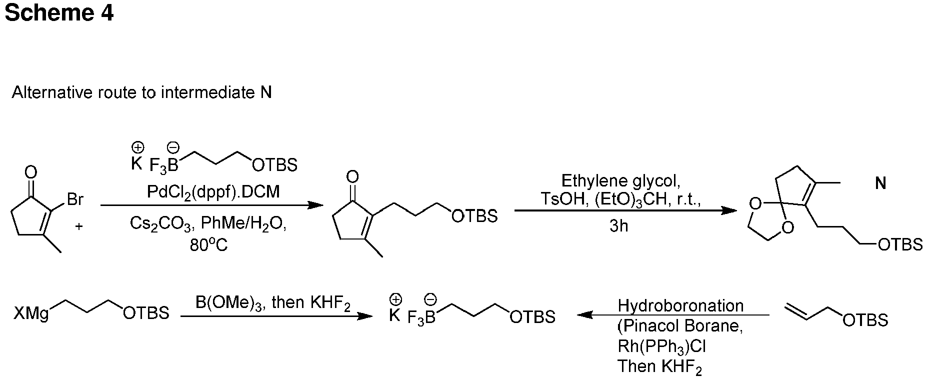

In Scheme 4, 2-bromo methyl cyclopentenone is converted to tert-butyldimethyl(3-(7-methyl-1,4-dioxaspiro[4.4]non-6-en-6-yl)propoxy)silane (Intermediate N) by converting the bromine group to the propylsilane group using a boron reagent (See, Molander, G. A.; Ham, J.; Seapy, D. G. Tetrahedron, 2007, 63, 768-775); which is followed by glycolization of the ketone.

In Scheme 5, shown below, intermediate N is converted to the hydroxyl intermediate (intermediate O). Intermediate O is then converted to one of three intermediates: 3-(7-methyl-1,4-dioxaspiro[4.4]non-6-en-6-yl)propanal (intermediate S), or an intermediate having a bulky phosphorous or silicon group: Intermediate Q or Intermediate R; each of which may be utilized in the next phase of the reaction.

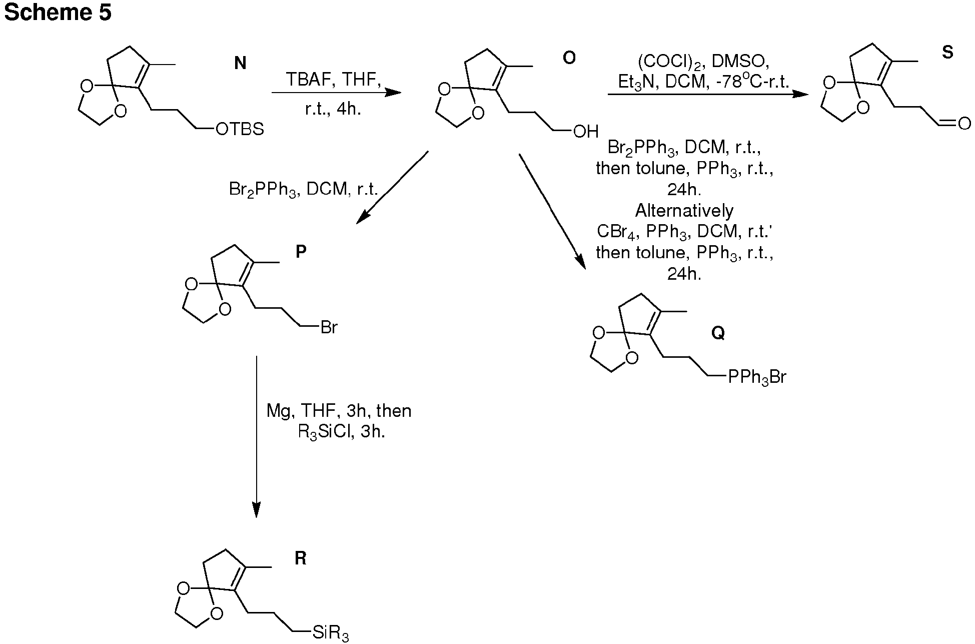

In scheme 6, shown below, 3-methyl-2-((3E,7E)-7-methyltrideca-3,7-dien-11-ynyl)cyclopent-2-enone (intermediate T) may be produced by one of two reaction approaches.

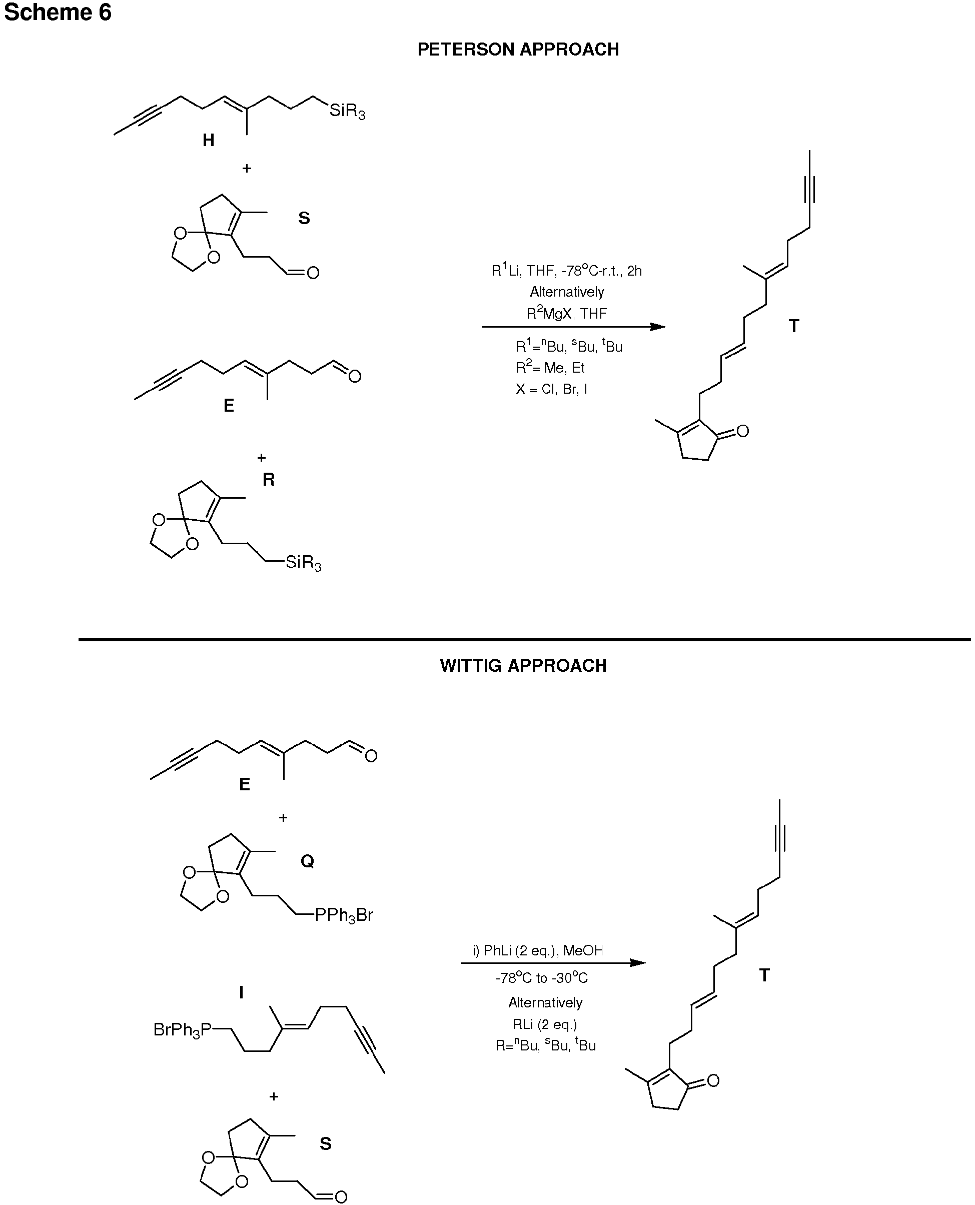

In the Peterson approach, W. Adam, C. M. Ortega-Schulte, Synlett, 2003, 414-416 and A. Barbero, Y. Blanco, C. Garcia, Synthesis, 2000, 1223-1228, which is incorporated herein by reference in its entirety, Intermediates H and S or Intermediates E and R are reacted in the presence of s-butyl lithium to produce Intermediate T. This represents a new method in the synthesis of progesterones

In the Wittig approach, Johnson, W. S.; Gravestock, M. B.; McCarry, B. E. J. Am. Chem. Soc., 1971, 93, 4332, which is incorporated herein by reference in its entirety, Intermediates E and Q or Intermediates I and S are reacted in the presence of phenyl lithium to produce Intermediate T.

In the final phase of the synthesis, shown in Scheme 7 shown below, Intermediate T is cyclized to form a racemic mixture of 1-((1R,3aR,3bR,8aS,8bR,10aR)-6,8a,10a-trimethyl-1,2,3,3a,3b,4,5,7,8,8a,8b,9,10,10a-tetradecahydrodicyclopenta[a,f]naphthalen-1-yl)ethanone and 1-((1S,3aS,3bS,8aR,8bS,10aS)-6,8a,10a-trimethyl-1,2,3,3a,3b,4,5,7,8,8a,8b,9,10,10a-tetradecahydrodicyclopenta[a,f]naphthalen-1-yl)ethan-1-one (Intermediate U, one enantiomer shown).

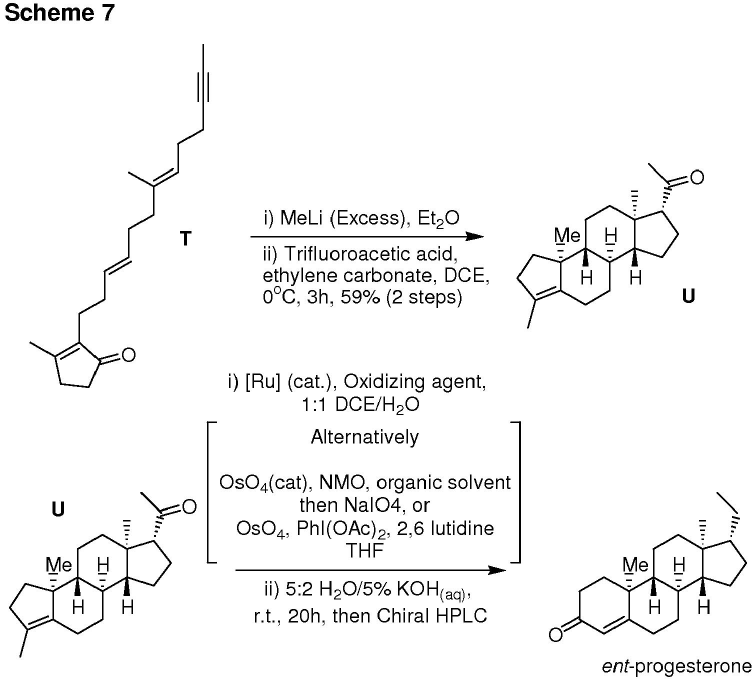

Intermediate U is then reacted in dichloroethene in the presence of a catalytic amount of ruthenium(III) chloride and sodium periodate followed by treatment with potassium hydroxide in water to form the final chiral product, ent-progesterone.

As described above, an enantiomerically-enriched ent-progesterone may be obtained by separation of enatiomers, either of a racemic intermediate or racemic progesterone. Thus, the present invention further contemplates a method of preparing ent-progesterone by isolating ent-progesterone from racemic progesterone. The present invention also contemplates preparing ent-progesterone by reacting an enantiomerically-enriched intermediate, e.g., intermediate U disclosed herein, and transforming the enantiomerically-enriched intermediate through one or more reaction steps to provide ent-progesterone.

Separation of enantiomerically-enriched compounds, e.g., intermediates or progesterone, from a racemic mixture may be performed accroding to a variety of methods some of which are known in the art. For example, chiral high performance liquid chromatography (HPLC) may be used to separate enantiomers. HPLC columns having chiral stationary phases suitable for chiral HPLC are commercially available. Alternatively, enantiomers may be separated by methods such as (i) recrystallization or complexation with a chiral material, followed by isolation of the enantiomer; (ii) derivatization with a chiral auxiliary and separation of diastereomers, followed by cleavage of the auxiliary and recovery of the enantiomer; (iii) resolution by selective reaction with an enantiomerically-enriched reagent, e.g., an enzyme or a chiral reduction of oxidation reagent, that modifies one enantiomer while leaving the other enantiomer substantially unchanged, followed by separation of the desired enantiomer.

Prior to the inventive method, the preparation of ent-progesterone from Intermediate U required the use of a dangerous and costly ozonolysis step. The inventive method of the present invention utilizes readily available materials and results in a compound having about >98% purity.

### Examples

### Abbreviations and Acronyms

A comprehensive list of the abbreviations used by organic chemists of ordinary skill in the art appears in The ACS Style Guide (third edition) or the Guidelines for Authors for the Journal of Organic Chemistry. The abbreviations contained in said lists, and all abbreviations utilized by organic chemists of ordinary skill in the art are hereby incorporated by reference. For purposes of this invention, the chemical elements are identified in accordance with the Periodic Table of the Elements, CAS version, Handbook of Chemistry and Physics, 67th Ed., 1986-87, each of which is incorporated herein by reference in its entirety.

More specifically, when the following abbreviations are used throughout this disclosure, they have the following meanings:
- atm: atmosphere
- br s: broad singlet
- Buchi: rotary evaporator ®BÜCHI Labortechnik AG
- C: Celsius
- CDCl₃: deuterated trichloromethane
- Celite: diatomaceous earth filter agent ®Celite Corp.
- d: doublet
- dd: doublet of doublets
- DIBAL-H: diisobutylaluminum hydride
- DCM: dichloromethane
- DMI: dimethyl-2-imidazolidinone
- g: gram
- h: hour, hours
- ¹H NMR: proton nuclear magnetic resonance
- HPLC: high performance liquid chromatography
- *J*: coupling constant (NMR spectroscopy)
- L: liter
- LAH: lithium aluminum hydride
- LG: leaving group
- M: mol L-1 (molar)
- m: multiplet
- MHz: megahertz
- min: minute, minutes
- mL: milliliter
- µM: micromolar
- mol: mole
- MS: mass spectrum, mass spectrometry
- m/z: mass-to-charge ratio
- N: equivalents L-1 (normal)
- NBS: N-bromo succinimide
- NMO: N-Methylmorpholine-N-Oxide
- NMR: Nuclear Magentic Resonance
- pH: negative logarithm of hydrogen ion concentration
- q: quartet
- RBF: round bottom flask
- r.t: room temperature
- RT: retention time (HPLC)
- rt: room temperature
- s: singlet
- t: triplet
- THF: tetrahydrofuran
- TLC: thin layer chromatography
- TsCl: tosyl chloride

The percentage yields reported in the following examples are based on the starting components that are used in the lowest molar amount. Air and moisture sensitive liquids and solutions are transferred via syringe or cannula, and are introduced into reaction vessels through rubber septa. Commercial grade reagents and solvents are used without further purification. The term "concentrated under reduced pressure" refers to use of a Buchi rotary evaporator at 15 mm of Hg. All temperatures are reported uncorrected in degrees Celsius (°C). Thin layer chromatography (TLC) is performed on pre-coated glass-backed silica gel 60 A F-254 250 µm plates.

The structures of compounds of this invention are confirmed using one or more of the following procedures.

### NMR

NMR spectra are acquired for each compound when indicated in the procedures below. NMR spectra obtained were consistent with the structures shown.

Routine one-dimensional NMR spectroscopy was performed on either 300 or 500 MHz Varian® Mercury-plus spectrometers. The samples were dissolved in deuterated solvents. Chemical shifts were recorded on the ppm scale and were referenced to the appropriate solvent signals, such as 2.49 ppm for DMSO-d6, 1.93 ppm for CD3CN, 3.30 ppm for CD3OD, 5.32 ppm for CD2Cl2 and 7.26 ppm for CDCI3 for 1H spectra.

### Materials

A VWR Dyastir magnetic stirrer is used for all reactions. Pyrex® brand glassware is used unless otherwise stated. Chemicals and solvents that are used in the experimental workups are purchased from Sigma Aldrich, Fisher Scientific or EMD unless otherwise stated and the solvents used are either ACS or HPLC grade with the two grades being used interchangeably. For TLC analysis, the silica 60 gel glass backed TLC plates are purchased from EMD.

### Synthesis of Intermediate A

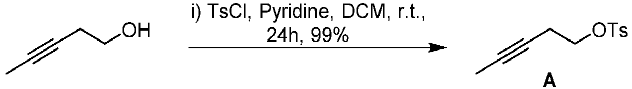

Compound **A** was prepared according to the method of Battenberg, O. A.; Nodwell, M. B.; Sieber, S. A. J. Org. Chem., 2011, 76, 6075-6087. To a dried, 1 L round bottom flask (RBF), equipped with a stirrer bar, under an atmosphere of Argon was added 250 mL of ACS grade dichloromethane (DCM) (Fisher Chemicals), 18.5 mL, 200 mmol, of 3-pentyn-1-ol (Sigma-Aldrich), 76 g, 400 mmol of tolunesulfonyl chloride (TsCl) and 45 mL of pyridine (Fisher Chemicals) sequentially. The reaction was then stirred for 18 h and was monitored by thin layer chromatography (TLC). After TLC analysis indicates the reaction has gone to completion the reaction mixture was quenched with 200 mL of a saturated, aqueous copper sulfate solution. The biphasic mixture was vigorously shaken and separated using a 1 L separatory funnel. The organic phase was collected and the aqueous phase was further extracted with two 75 mL portions of DCM. The combined organic phases are then washed with a sodium hydrogen carbonate (NaHCO₃) and the aqueous layer was separated and extracted as before with two 75 mL portions of DCM. The combined organic phases are dried with sodium sulfate and filtered through a 250 mL sinter funnel into a 1 L mL RBF. The filtered residue was washed with a further 100 mL of DCM and the collected solution in the RBF was reduced under vacuum on a rotary evaporator (Buchi) to give Compound **A** as a clear oil. The proton nuclear magnetic resonance (NMR) spectrum in deuterated chloroform (CDCl₃) matched the previously reported data. (See, Fang, F.; Vogel, M.; Hines, J. V.; Bergmeier, S. C.; Org. Biomol. Chem., 2012, 10, 3080-3091.)

### Synthesis of Intermediate B

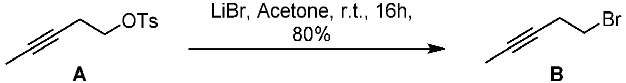

Compound **B** was prepared according to the method of Snider, B. B.; Kirk, T. C.; J. Am. Chem. Soc., 1983, 105, 2364-2368. To a dried 500 mL RBF, equipped with a stirrer bar, under an atmosphere of Argon was added 200 mL of ACS grade acetone (Fisher Chemicals) and 48 g, 200 mmol, of Compound **A.** The solution was stirred vigorously and cooled to 0°C with an ice bath whereupon 35 g of lithium bromide was added portion-wise over 5 minutes. The ice bath was removed after a further 10 minutes and the reaction allowed to warm to room temperature where it was stirred for a further 24 hours. After TLC analysis indicates the reaction has gone to completion the reaction mixture was diluted with 200 mL of hexane (EMI) and the mixture was filtered through a 250 mL sinter funnel with a 1 inch plug of celite (Sigma-Aldrich) into a 500 mL RBF. The collected filtrate was then reduced under vacuum on a rotary evaporator (Buchi) to give Compound **B** as a clear oil. If a white precipitate was present the crude product was redissolved in hexane and the workup procedure repeated. The proton nuclear magnetic resonance (NMR) spectrum in CDCl₃ matched the previously reported data. (See, Lubell, W. D.; Jamison, T. F.; Rapoport, H. J. Org. Chem., 1990, 55, 3511-3522.)

### Synthesis of Intermediate C

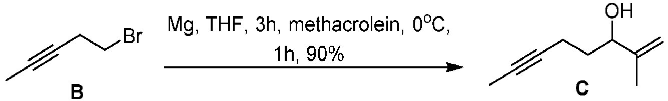

Compound C was prepared according to the method of Johnson, W. S.; Gravestock, M. B.; McCarry, B. E. J. Am. Chem. Soc., 1971, 93, 4332-4334. To a dried 500 mL RBF, equipped with a stirrer bar, under an atmosphere of Argon was added 120 mL of distilled THF, followed by 0.62 g, 31 mmol, of magnesium turnings (Sigma Aldrich) and the mixture was vigorously stirred at room temperature. Compound **B** (4.41 g, 30 mmol) was the added to the flask via syringe in one portion and the reaction mixture was stirred at room temperature for 3 hours or until most of the magnesium has been consumed, whereupon the reaction mixture was cooled with an ice bath to 0°C. Meanwhile, in a separate, dried, 25 mL RBF 2.56 mL, 31 mmol, of methacolein in 10 mL of distilled THF was cooled to 0°C with an ice bath. The methacrolein solution was then added to the Grignard solution via cannula over 10 minutes. The reaction mixture was then allowed to warm to room temperature and left for 1 hour. The reaction mixture was subsequently quenched with 75 mL of saturated, aqueous ammonium chloride solution and diluted with 150 mL of ethyl acetate. After being vigorously shaken, the biphasic mixture was then separated with a separatory funnel and the aqueous phase was further extracted with two 75 mL portions of ethyl acetate. The combined organic phases are then dried with sodium sulfate and filtered through a plug of 1 inch of Celite and 1 inch of flash silica (silica gel 60, EMD) via a 100 mL sinter funnel under vacuum into a 1 L RBF, with the sodium sulfate residue washed with a further 75 mL of ethyl acetate. The collected solution was then reduced under vacuum on a Buchi rotary evaporator to give compound **C** as a light yellow oil in 90% yield and >95% purity. The proton NMR spectrum in CDCl₃ agreed with the previously reported data. (See, Apparu, M.; Barrelle, M. Bulletin de la Societe Chimique de France, 1983, 3-4, Pt. 2, 83-86).

### Synthesis of Intermediate D

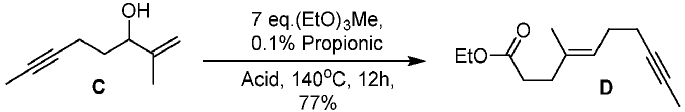

Compound **D** was prepared according to the method of Johnson, W. S.; Gravestock, M. B.; McCarry, B. E. J. Am. Chem. Soc., 1971, 93, 4332-4334. To a dried 150 mL RBF, equipped with a stirrer bar, under an atmosphere of Argon was added a solution of 4.14 g, 30 mmol, of Compound **C** in 7 molar equivalents of trimethylorthoacetate (Sigma-Aldrich) via syringe followed by 1 mol % of propionic acid (Sigma-Aldrich). The reaction vessel was fitted with a reflux condenser and the mixture was then heated to reflux with a 1200 mL Instatherm® oil bath for 12 hours. The reaction was then removed from the oil bath and allowed to cool to room temperature. The crude product mixture was washed with a saturated sodium hydrogen carbonate solution (100 mL) and the aqueous layer removed via a 1 L separatory funnel, before being further extracted with 100 mL of ethyl acetate which was subsequently combined with the product mixture and reduced under vacuum on a Buchi rotary evaporator. Purification via short path distillation under reduced pressure gave Compound **D** as a clear oil in 71% yield and >95 purity. **1H NMR (500 MHz, CDCl3): δ = 5.19 (tq, J = 6.8, 1.2, 1H), 4.10 (q, J = 7.2, 2H), 2.44-2.35 (m, 2H), 2.32-2.27 (m, 2H), 2.18-2.08 (m, 4H), 1.76 (t, J** = **2.4, 3H), 1.61 (bs, 3H), 1.23 (t, J = 7.10, 3H).**

### Synthesis of Intermediate E

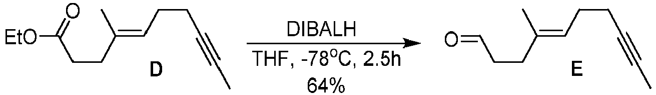

Compound **E** was prepared according to the method of Johnson, W. S.; Gravestock, M. B.; McCarry, B. E. J. Am. Chem. Soc., 1971, 93, 4332-4334. To a dried 150 mL RBF, equipped with a stirrer bar, under an atmosphere of Argon was added 50 mL of distilled THF and 2.08 g, 10 mmol, of Compound **D** and the mixture was cooled to -78°C in a dry-ice/acetone bath. After 15 minutes 12 mL, 12 mmol, of a 1 M solution of diisobutylaluminum hydride (DIBAL-H) in THF was added over 10 minutes and the reaction mixture was then left to stir for 2.5 hours. The reaction was then quenched with 5 mL of methanol at -78°C over 10 minutes and then allowed to warm to room temperature before 20 mL of water was added. The reaction mixture was extracted with 100 mL of ethyl acetate via a 1 L separatory funnel and the aqueous phase was further extracted with 2 more 50 mL portions of ethyl acetate and the combined extracts are dried with 100 g of sodium sulfate and reduced under vacuum on a Buchi rotary evaporator to give the crude product, Compound **E,** as a light yellow oil. Purification by flash column chromatography (Silica gel 60, EMD, 10:1 hexane/ethyl acetate) gave Compound **E** as a clear oil in 64% yield and >95% purity. **1H NMR (300 MHz, CDCl3): δ = 9.75 (t, J = 1.8, 1H), 5.20 (m, 1H), 2.52 (tm, J = 7.5, 2H), 2.32 (t, J = 7.5, 2H), 2.22-2.07 (m, 4H), 1.76 (t, J** = **2.4, 3H), 1.62 (bs, 3H).**

### Synthesis of Intermediate F

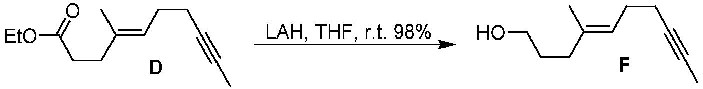

Compound **F** was prepared according to the method of Johnson, W. S.; Gravestock, M. B.; McCarry, B. E. J. Am. Chem. Soc., 1971, 93, 4332-4334. To a dried 1 L RBF, equipped with a stirrer bar, under an atmosphere of argon was added 250 mL of ether followed by 3.42 g, 90 mmol, of Lithium Aluminum Hydride (LAH, Sigma-Aldrich). The mixture was cooled to 0°C with an ice bath and after 15 minutes Compound **D** (9.0 g, 45 mmol), dissolved in 50 mL of ether was added over 10 minutes. After a further 1 hour or when TLC analysis indicates the reaction has gone to completion the reaction was quenched with 100 mL of 10% w/w aqueous sodium hydroxide solution over 5 minutes and then 50 mL of water before being brought to room temperature. The reaction mixture was extracted with 100 mL of ethyl acetate and the aqueous phase further with ethyl acetate (2 x 100 mL) utilizing a 1 L separatory funnel. The combined organic phases are dried with 50 g of sodium sulfate, filtered through a 100 mL sinter funnel, and reduced under vacuum on a rotary evaporator (Buchi) to give the crude product, Compound **F**, as a clear oil. Purification by flash column chromatography (Silica gel 60, EMD, 5:1 hexane/ethyl acetate) gave Compound F as a clear oil in 92% yield and >95% purity. **¹H NMR (500 MHz, CDCl₃): δ = 5.22 (t, *J* = 6.8, 1H), 3.64 (t, *J* = 6.4, 2H), 2.21-2.11 (m, 4H), 2.08 (t, *J* = 7.5, 2H), 1.77 (bs, 3H), 1.68 (tt, *J* = 6.9, 6.9, 2H), 1.63 (s, 3H).**

### Synthesis of Intermediate G

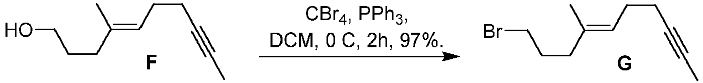

Compound **G** was prepared according to the method of Baughman, T. W.; Sworen, J. C.; Wagener, K. B. Tetrahedron, 2004, 60, 10943-10948. To a dried RBF, equipped with a stirrer bar, under an atmosphere of Argon was added 35 mL of DCM followed by 3.88 g carbon tetrabromide (Sigma-Aldrich) and 2.56 g of triphenyl phosphine (Sigma-Aldrich). The reaction mixture was cooled to 0°C with an ice bath and after 15 minutes 1.06 g of Compound **F**, dissolved in 10 mL of DCM was added over 5 minutes. After a further 2 hours or when TLC analysis indicates the reaction has gone to completion the reaction was diluted with 100 mL of hexane and filtered through 1 inch of Celite via a 100 mL sinter funnel into a 500 mL RBF. The solution was reduced under vacuum on a Buchi rotary evaporator to give Compound **G** as a clear oil. If a white precipitate was present the crude product was redissolved in hexane and filtered through a plug of 1 inch of Celite above 1 inch of flash silica (silica gel 60, EMD) and reduced under vacuum to give Compound G as a clear oil in 97% yield and >95% purity. **¹H NMR (500 MHz, CDCl₃): δ = 5.21 (t, *J* = 6.8, 1H), 3.38 (t, *J* = 6.8, 2H), 2.20-2.11 (m, 6H), 1.98-1.90 (m, 2H), 1.77 (bs, 3H), 1.61 (s, 3H).**

### Synthesis of Intermediate H

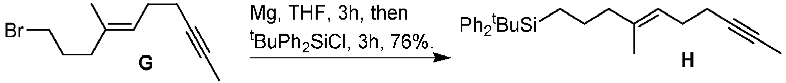

Compound H was prepared according to the method of Dixon, T. A.; Steele, K. P.; Weber, W. P. J. Organomet. Chem. 1982, 231, 299-305. To a dried 100 mL RBF, equipped with a stirrer bar, under an atmosphere of Argon was added 20 mL of distilled THF, followed by 50 mg of magnesium turnings (Sigma-Aldrich) and the mixture was vigorously stirred at room temperature. Compound **G** (0.46 g, 2 mmol) in 5 mL of distilled THF was the added to the flask via syringe in one portion and the reaction mixture was stirred at room temperature for 3 hours or until most of the magnesium has been consumed, whereupon 0.5 mL, 2 mmol, of *tert-*butyldiphenylsilyl chloride, dissolved in 5 mL of distilled THF, was added in one portion via syringe and the reaction was left to stir at room temperature for a further 3 hours. The reaction mixture was subsequently quenched with 50 mL of saturated, aqueous ammonium chloride solution and diluted with 100 mL of ethyl acetate and transferred to separatory funnel. After being vigorously shaken, the biphasic mixture was then separated and the aqueous phase was further extracted with two 50 mL portions of ethyl acetate. The combined organic phases are then dried with sodium sulfate and filtered through a plug of 1 inch of Celite and 1 inch of flash silica (silica gel 60, EMD) via a 100 mL sinter funnel under vacuum into a 500 mL RBF, with the sodium sulfate residue washed with a further 50 mL of ethyl acetate. The collected solution was then reduced under vacuum on a Buchi rotary evaporator to give compound **H,** crude, as a clear oil. Purification by flash column chromatography (Silica gel 60, EMD, hexane) gave Compound **H** as a clear oil in 76% yield and > 95% purity. **1H NMR (300 MHz, CDCl3): δ = 7.79-7.73 (m, 4H), 7.49-7.36 (m, 6H), 5.16 (t, J = 6.3, 1H), 2.24-2.09 (m, 4H), 1.96 (t, J = 7.5, 2H), 1.78 (t, J = 2.4, 3H), 1.60 (bs, 3H), 1.48-1.33 (m, 2H), 1.14 (s, 9H), 0.87 (t, J = 7.2, 2H).**

### Synthesis of Intermediate I

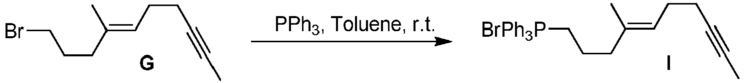

Compound **I** may be prepared by adapting the method of Byrne, P. A.; Gilheany, D. G. J. Am. Chem. Soc., 2012, 134, 9225-9239. To a dried 250 mL RBF under an argon atmosphere is added 100 mL of distilled toluene, followed by 2.28 g, 10 mmol, of Compound **G,** and 5.27 g, 20 mmol, of triphenylphosphine (Sigma-Aldrich) and the reaction is stirred at room temperature and monitored by TLC analysis. Upon completion the reaction mixture is reduced under vacuum on a Buchi rotary evaporator. The residue is taken up in 5:1 hexane/ethyl acetate and purified by flash column chromatography (Silica gel 60, EMD, hexane) to give Compound **I**, expected to be a white solid.

### Synthesis of Intermediate J

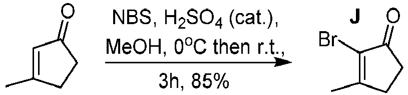

Compound **J** was prepared according to the method of Bliese, M.; Cristiano, D.; Tsanaktsidis, J. Aust. J. Chem., 1997, 50, 1043-1045. To a dried 1 L RBF, equipped with a stirrer bar, under an atmosphere of Argon was added 60 mL of methanol (Aldrich, HPLC grade) followed by 0.99 mL, 10 mmol, of 3-methyl cyclopentenone and 1.762 g, 9.9 mmol, of N-bromo succinimide. The reaction mixture was cooled to 0°C with an ice bath over 15 minutes, whereupon conc. sulfuric acid (0.2 eq.) was added and the reaction was left to stir for 3 hours being allowed to warm to room temperature over this time. Subsequently 50 mL of saturated sodium hydrogen carbonate and 40 mL of DCM are added and the mixture was transferred to a separatory funnel. After being vigorously shaken, the biphasic mixture was then separated and the aqueous phase was further extracted with two 50 mL portions of DCM. The combined organic phases are then dried with sodium sulfate and filtered through a plug of 1 inch of Celite and 1 inch of flash silica (silica gel 60, EMD) via a 100 mL sinter funnel under vacuum into a 500 mL RBF, with the sodium sulfate residue washed with a further 50 mL of DCM. The collected solution was then reduced under vacuum on a Buchi rotary evaporator to give compound **J,** crude, as a light yellow solid. Purification by flash column chromatography (Silica gel 60, EMD, hexane) gave Compound **J** as a cream crystalline in 85% yield and >98% purity. The ¹H NMR spectrum in CDCl₃ agreed with the previously reported data. (see, Bliese, M.; Cristiano, D.; Tsanaktsidis, J. Aust. J. Chem., 1997, 50, 1043-1045.)

### Synthesis of Intermediate K

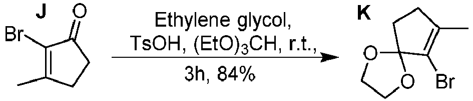

Compound **K** was prepared according to the method of Richter, A.; Hedberg, C.; Waldmann, H. J. Org. Chem., 2011, 76, 6694-6702. To a 500 mL rbf, equipped with a stirrer bar, under an atmosphere of Argon was added 200 mL of triethyl orthoacetate (Aldrich), 7.8 g, 40 mmol, of Compound **J** and 38 mg, 0.2 mmol of *para*toluenesulfonic acid. The reaction mixture was stirred at room temperature for 3 hours or until TLC analysis indicates the reaction has gone to completion and the product was distilled off under vacuum to give Compound **K** as a clear oil which solidified to a white solid on cooling in 88% yield >96% purity. The ¹H NMR spectrum in CDCl₃ agreed with the previously reported data. (See, Richter, A.; Hedberg, C.; Waldmann, H. J. Org. Chem., 2011, 76, 6694-6702.)

### Synthesis of Intermediate L

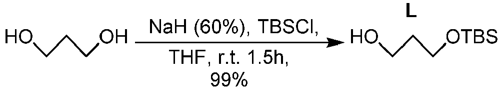

Compound **L** was prepared according to the procedure of McDougal, P. G.; Rico, J. G.; Oh, Y.-I.; Condon, B. J. Org. Chem., 1986, 51, 3388-3390. To a dried 250 mL RBF under an atmosphere of argon at room temperature was added 100 mL of distilled THF and 2.1 g of sodium hydride (60% dispersion in mineral oil; Aldrich). The mixture was stirred vigorously and 1,3-propanediol (4.0 g, 50 mmol; Aldrich) was added over 10 minutes via syringe. The reaction was allowed to stir for 45 minutes before tert-butyldimethylsilyl chloride (7.9 g, 52.7 mmol; Aldrich) was added portion wise over 5 minutes. The reaction was then allowed to stir for a further 45 minutes at room temperature before being quenched slowly with 20 mL of 10% aqueous sodium carbonate solution. This mixture was then transferred to a separatory funnel. After being vigorously shaken, the biphasic mixture was separated and the aqueous phase was further extracted with two 50 mL portions of ether. The combined organic phases are then dried with sodium sulfate and filtered through a plug of 1 inch of Celite and 1 inch of flash silica (silica gel 60, EMD) via a 100 mL sinter funnel under vacuum into a 500 mL RBF, with the sodium sulfate residue washed with a further 50 mL of ether. The collected solution was then reduced under vacuum on a Buchi rotary evaporator to give compound **L**, as a light yellow oil in 99% yield and >95% purity. The ¹H NMR spectrum in CDCl₃ agreed with the previously reported data. (See, McDougal, P. G.; Rico, J. G.; Oh, Y.-I.; Condon, B. J. Org. Chem., 1986, 51, 3388-3390.)

### Synthesis of Intermediate M

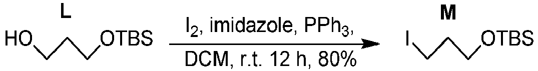

Compound **M** was prepared according to the procedure of Jakobsche, C. E.; Peris, G.; Miller, S. J. Angew. Chemie., Int. Ed., 2008, 47, 6707. To a dried 100 mL RBF under an atmosphere of argon at room temperature was added 25 mL of HPLC grade DCM, 0.81 g (5 mmol) of Compound **L,** 0.37 g (5.5 mmol) of imidazole (Aldrich), 1.45 g (5.5 mmol) of triphenylphosphine (Aldrich) and 1.4 g (5.5 mmol) of iodine (Fisher Chemicals). The reaction mixture was then stirred at room temperature for 12 hours, after which time it was diluted with hexane (100 mL) and filtered through a plug of 1 inch of Celite and 2 inches of flash silica (silica gel 60, EMD) via a 100 mL sinter funnel under vacuum into a 500 mL RBF. The collected solution was then reduced under vacuum on a Buchi rotary evaporator to give compound **M**, as a light clear oil in 80% yield and >95% purity. Residual triphenylphosphine may be removed by re-dissolving the product in hexane and filtering through another Celite/silica plug as described above. The ¹H NMR spectrum in CDCl₃ agreed with the previously reported data. (See, Jakobsche, C. E.; Peris, G.; Miller, S. J. Angew. Chemie., Int. Ed., 2008, 47, 6707.)

### Synthesis of Intermediate N

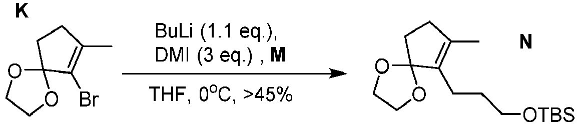

Compound **N** was prepared according to the procedure of Smith III, A. B.; Branca, S. J.; Pilla, N. N.; Guaciaro, M. A. J. Org. Chem., 1982, 47, 1855-1869, adapted with HMPA substituted for DMI. (see: Lo, C.-C.; Chao, P.-M. J. Chem. Ecology., 1990, 16, 3245-3253.) To a dried 100 mL RBF, equipped with a stirrer bar, under an atmosphere of argon was added 25 mL of distilled THF which was then cooled to -78°C with a dry ice bath. Then 3.44 mL, 5.5 mmol, of 1.6 M solution n-Butyllithium in hexanes (Aldrich) was added via syringe and the solution was allowed to stir for a further 15 minutes. Compound **K** (1.1 g, 5 mmol) was then added in 5 mL of distilled THF over 5 minutes and the reaction was allowed to stir at -78°C for a further 1 hour. After this time 3 equivalents of 1,3-Dimethyl-2-imidazolidinone (DMI), (1.71 mL, 15 mmol), was added drop-wise to the reaction mixture followed by, 30 minutes later, 1.36 g, 5 mmol, of Compound **M** dissolved in 5 mL of THF which was added over 10 minutes. The reaction was then left to stir until TLC analysis indicates complete consumption of the starting material during which time it was allowed to warm to -55°C and subsequently quenched with 25 mL of a saturated aqueous sodium dihydrogen phosphate solution. The reaction mixture then warmed to room temperature and diluted with 75 mL of ether and the mixture was transferred to a separatory funnel. After being vigorously shaken, the biphasic mixture was then separated and the aqueous phase was further extracted with two 50 mL portions of ether. The combined organic phases are then dried with sodium sulfate and filtered through a plug of 1 inch of Celite and 1 inch of flash silica (silica gel 60, EMD) via a 100 mL sinter funnel under vacuum into a 500 mL RBF, with the sodium sulfate residue washed with a further 50 mL of ethyl acetate. The collected solution was then reduced under vacuum on a Buchi rotary evaporator to give compound **N,** crude, as a light yellow oil. Purification by flash column chromatography (Silica gel 60, EMD, 5:1 hexane/ethyl acetate) gave Compound **N** as a light yellow oil in 45% yield and > 98% purity by GC-MS. GC-MS: 10.57 min, m[H]⁺ = 313.2.

### Combined Synthesis of Intermediate N

An alternative synthesis of compound **N,** which may be referred to as a "combined synthesis," is shown below in Scheme 8.

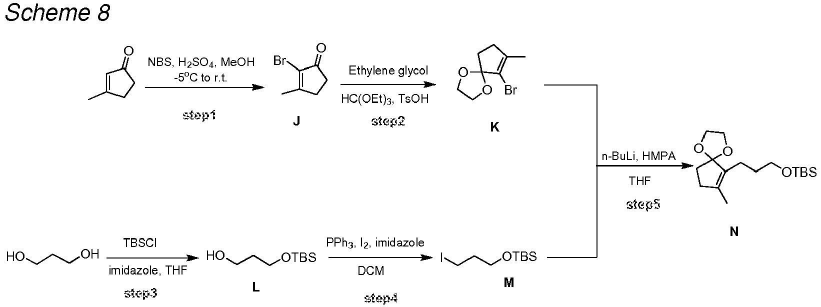

A detailed description of each of the five steps of Scheme 8 is provided below.

### (Step 1)

Under nitrogen atmosphere, charge 3-Methyl-2-cyclopenten-1-one (1.0 eq) and MeOH (6.0 v) to the reactor with stirring. Batchwise charge NBS (0.99 eq) at 15-25 °C, then charge con.H₂SO₄ (0.02eq) below 5 °C. Stir the system at 15∼25 °C until the reaction completed shown on TLC. Charge sat.NaHCO₃ (6.0 v) and DCM (4.0 v) to the system and stir for 10 mins. Separate and extract the water layer with DCM (2.0 v) twice. Combine the organic layer and wash with brine (6.0 v). Separate and collect the organic layer. Charge con.HCl (2.5 v) to the organic layer and stir for 20 hrs at r.t., separate and extract the water layer with DCM (2.0 v) twice. Combine the organic layer and wash with brine (6.0 v). Dry the organic layer with Na₂SO₄. Filter and concentrate the filtrate under vacuum at 30-35 °C. The residue recrystallize in PE/EA=0.8 v/1.2 v to give solid product of Intermediate J. The yield was 85%.

### (Step 2)

Charge Intermediate J (1.0 eq), triethyl orthoformate(3.5 eq), glycol (7.0 eq) and TsOH (0.01 eq) to reactor under N₂. Stir at 20∼25°C for 16 hrs. Charge sat.NaHCO₃ (5.0 v) and cyclohexane (4.0 v) to the system. Stir for 10 min and separate. Extract the water layer with cyclohexane (3.0 v) twice and combine the organic layer. Wash the organic layer with brine (4.0 v). Dry the organic layer with Na₂SO₄. Filter and concentrate the filtrate under vacuum. Distill the residue under 5 mmHg to get the product of Intermediate K. The yield was 88%.

### (Step 3)

Charge propanediol (4.0 eq), THF (8.0 v) and imidazole (1.0 eq) to reactor. Charge TBSCI (1.0 eq) dropwise at -2∼2°C. stir at -2∼2°C for 2 hrs and then 20∼25 °C for 3 hrs. Charge water (10.0 v) and EA (5.0 v) to system. Stir for 10 mins and separate. Extract the water layer with EA (2.0 v) twice and combine the organic layer. Wash the organic layer with brine (4.0 v) and dry with Na₂SO₄. Filter and concentrate the filtrate under vacuum to give the crude product of Intermediate L used directly for next step.

### (Step 4)

Charge crude Intermediate L (1.0 eq), DCM (10.0 v), imidazole (1.5 eq) and PPh₃ (1.5 eq) to reactor. Charge I₂ (1.5 eq) at 0∼5 °C. stir at 0∼5 °C for 0.5 hrs then 20∼25 °C for 0.5 hrs. Charge water (5.0 v) to system and stir for 10 mins. Separate and wash the organic layer with brine (5.0 v) twice. Dry the organic layer with Na₂SO₄. Filter and concentrate the filtrate under vacuum. The residue was purified by column to give the oil product of Intermediate M. The yield for 2 steps was 80%.

### (Step 5)

Charge Intermediate K (1.0 eq) and THF (10.0 v) to reactor under N₂. Cool the system below -78 °C. Charge n-BuLi (1.5 eq) dropwise below -70 °C and stir for 1h. Charge HMPA (3.0 eq) dropwise below -65 °C and stir for 0.5 hrs. Charge PH-PRV-1301-102 (1.0 eq) dropwise below -65 °C and stir for 5 hrs at -60∼-50 °C. Charge water (20.0 v) and EA (5.0 v). Stir for 10 mins and separate. Extract the water layer with EA (2.0 v) twice and combine the organic layer. Wash the organic layer with brine (5.0 v). Dry the organic layer with Na₂SO₄. Filter and concentrate the filtrate under vacuum to give the crude product of Intermediate M (crude yield ∼= 96% and purity ∼=55%).

### Synthesis of Intermediate O

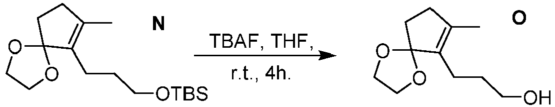

To a 500 mL RBF, equipped with a stirrer bar, under an atmosphere of argon at room temperature is added 150 mL of THF (ACS grade), 15.6 g, 50 mmol of Compound **N** and 100 mL of a 1 M solution of tetrabutylammonium fluoride (TBAF) in THF (Sigma-Aldrich). The reaction is stirred at room temperature for 4 hours or until TLC analysis indicates the reaction has gone to completion, whereupon 150 mL of water and 150 mL of ethyl acetate are sequentially added. This mixture is then transferred to a separatory funnel. After being vigorously shaken, the biphasic mixture is separated and the aqueous phase is further extracted with two 50 mL portions of ethyl acetate. The combined organic phases are then dried with sodium sulfate and filtered through a plug of 1 inch of Celite via a 100 mL sinter funnel under vacuum into a 500 mL RBF, with the sodium sulfate residue washed with a further 50 mL of ethyl acetate. The collected solution is then reduced under vacuum on a Buchi rotary evaporator to give compound **O.**

### Synthesis of Intermediate P

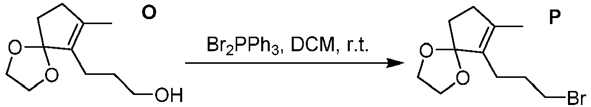

Compound **P** may be prepared by adapting the procedures of Lubell, W. D.; Jamison, T. F.; Rapoport, H. J. Org. Chem., 1990, 55, 3511-3522. To a 500 mL RBF, equipped with a stirrer bar, under an atmosphere of argon at room temperature is added 200 mL of distilled DCM, 9.9 g, 50 mmol of Compound **O** and 42.2 g, 100 mmol, of dibromotriphenylphosphorane (Sigma-Aldrich). The reaction mixture is stirred at room temperature and monitored by TLC analysis. An ice bath may be added at the beginning to prevent an exotherm. Once TLC analysis indicates the reaction has gone to completion the reaction mixture is filtered through a plug of 1 inch of Celite and 1 inch of flash silica (silica gel 60, EMD) via a 100 mL sinter funnel under vacuum into a 500 mL RBF. The collected solution is then reduced under vacuum on a Buchi rotary evaporator to give compound **P.** If a while precipitate is present the crude product is redissolved in hexane and filtered through a plug of 1 inch of Celite above 1 inch of flash silica (silica gel 60, EMD) and reduced under vacuum to give Compound **P.**

### Synthesis of Intermediate Q

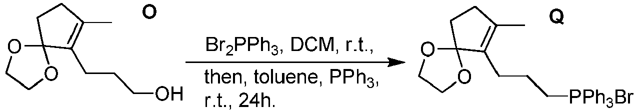

Compound **Q** may prepared by an adaptation of the procedures of Lubell, W. D.; Jamison, T. F.; Rapoport, H. J. Org. Chem., 1990, 55, 3511-3522 and Byrne, P. A.; Gilheany, D. G. J. Am. Chem. Soc., 2012, 134, 9225-9239.

To a 500 mL RBF, equipped with a stirrer bar, under an atmosphere of argon at room temperature is added 200 mL of distilled DCM, 9.9 g, 50 mmol of Compound **O** and 42.2 g, 100 mmol of dibromotriphenylphosphorane (Sigma-Aldrich). The reaction mixture is stirred at room temperature and monitored by TLC analysis. An ice bath may be added at the beginning to prevent an exotherm. Once TLC analysis indicates the reaction has gone to completion the reaction mixture is transferred directly to a Buchi rotary evaporator and reduced under vacuum. The residue is taken up in 200 mL of ACS grade toluene and 26.2 g, 100 mmol of triphenylphosphine is added. The reaction mixture is stirred for a further 24 hrs or until completion as indicated by TLC analysis. The reaction mixture is then transferred directly to a Buchi rotary evaporator and reduced under vacuum. The residue is then taken up in a 5:1 mixture of hexane/ethyl acetate and purified by flash column chromatography (Silica gel 60, EMD, hexane/ethyl acetate solvent system) to give Compound **Q.**

### Synthesis of Intermediate R

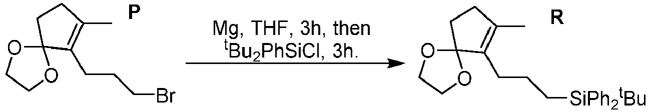

Compound **R** may be prepared by adapting the procedure of Dixon, T. A.; Steele, K. P.; Weber, W. P. J. Organomet. Chem. 1982, 231, 299-305. To a dried 250 mL RBF, equipped with a magnetic stirrer bar, under an argon atmosphere, is added 100 mL of distilled THF, followed by 0.48 g, 20 mmol, of magnesium turnings (Sigma-Aldrich) and the mixture is vigorously stirred at room temperature. Compound **P** (4.96 g, 19 mmol) in 10 mL of distilled THF is the added to the flask via syringe in one portion and the reaction mixture is stirred at room temperature for 3 hours or until most of the magnesium has been consumed, whereupon 4.47 mL, 21 mmol, of *tert*-butyldiphenylsilyl chloride dissolved in 10 mL of distilled THF is added in one portion via syringe and the reaction is left to stir at room temperature for a further 3 hours. The reaction mixture is subsequently quenched with 50 mL of saturated, aqueous ammonium chloride solution and diluted with 100 mL of ethyl acetate and transferred to separatory funnel. After being vigorously shaken, the biphasic mixture is then separated and the aqueous phase is further extracted with two 50 mL portions of ethyl acetate. The combined organic phases are then dried with sodium sulfate and filtered through a plug of 1 inch of Celite and 1 inch of flash silica (silica gel 60, EMD) via a 100 mL sinter funnel under vacuum into a 500 mL rbf, with the sodium sulfate residue washed with a further 50 mL of ethyl acetate. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give Compound **R.**

### Synthesis of Intermediate S

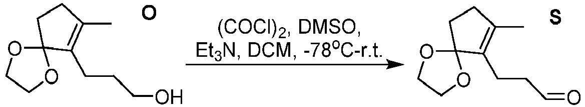

Compound **S** may be prepared by adapting the procedures of Miyata, O.; Muroya, K.; Kobayashi, T.; Yamanaka, R.; Kajisa, S.; Koide, J.; Naito, T. Tetrahedron, 2002, 58, 4459-4479. To a 250 mL RBF, equipped with a stirrer bar, under an atmosphere of argon is added 40 mL of distilled DCM and 0.77 mL, 9 mmol of oxalylchloride (Sigma-Aldrich) and the reaction mixture was cooled to -78°C with a dry ice bath. 1.25 mL, 17.6 mmol of dimethyl sulfoxide (Sigma-Aldrich) is then added drop wise by syringe and the reaction is stirred for a further 10 minutes. After this time a solution of 0.87 g, 4.5 mmol of compound **N,** dissolved in 10 mL of DCM, is added via syringe and the reaction is stirred for a further 15 minutes whereupon 2.5 mL of triethylamine (Sigma-Aldrich) is added over 5 minutes via syringe. The reaction is stirred for a further 15 minutes before being warmed to 0°C. After TLC analysis shows the reaction is completed the mixture is transferred directly onto a silica gel column (Silica gel 60, EMD) and the Compound S is isolated via flash chromatography (hexane/ethyl acetate solvent system).

### Synthesis of Intermediate T (From Intermediates E and Q)

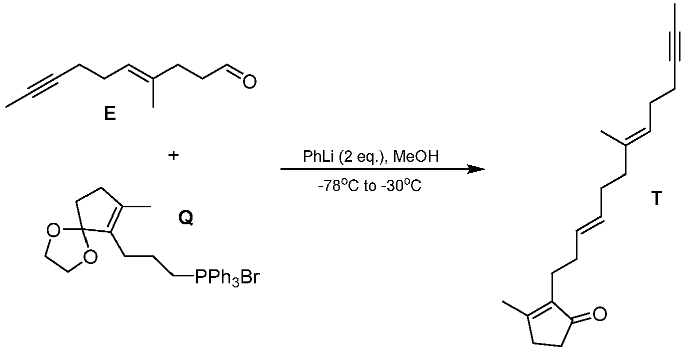

Compound **T** may be prepared by adapting the procedures of Johnson, W. S.; Gravestock, M. B.; McCarry, B. E. J. Am. Chem. Soc., 1971, 93, 4332-4334. To a dried 250 mL RBF under an atmosphere of argon at room temperature is added 100 mL of distilled THF, and 10.44 g, 20 mmol, of Compound Q. The resulting solution is then treated with 11.11 mL, 20 mmol, of 1.8 M phenyllithium in dibutyl ether (Sigma-Aldrich) and after 15 minutes is cooled to -78°C with a dry ice bath. After a further 15 minutes, 2.68 g, 20 mmol, of Compound **E** dissolved in 5 mL of dry THF is added via syringe and the reaction mixture is warmed to -30°C through transfer of the apparatus to a cryostat. A second equivalent of 1.8 M phenyllithium is then added followed by excess methanol with the temperature maintained at -30°C. After stirring for 5 minutes the reaction is brought to room temperature and 40 mL of water is added and the reaction mixture is transferred to a 1 L separatory funnel where 200 mL of ethyl acetate is added. After being vigorously shaken, the biphasic mixture is separated and the aqueous phase is further extracted with two 50 mL portions of ethyl acetate. The combined organic phases are then dried with sodium sulfate and filtered through a plug of 1 inch of Celite via a 100 mL sinter funnel under vacuum into a 1 L RBF, with the sodium sulfate residue washed with a further 50 mL of ether. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give Compound **T.**

### Synthesis of Intermediate T (From Intermediates I and S)

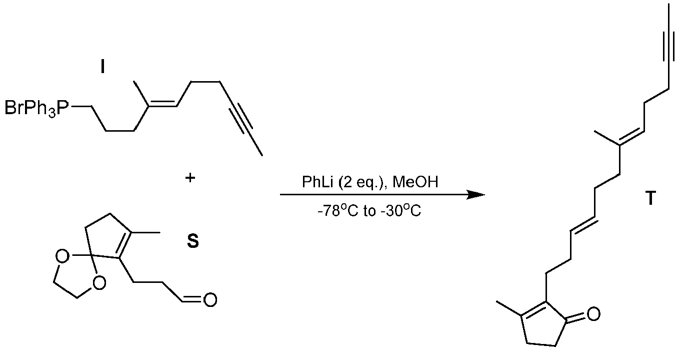

Compound **T** may also be prepared by adapting the procedures of Johnson, W. S.; Gravestock, M. B.; McCarry, B. E. J. Am. Chem. Soc., 1971, 93, 4332-4334 using different starting materials. To a dried 250 mL RBF under an atmosphere of argon at room temperature is added 100 mL of distilled THF, and 7.76 g, 20 mmol, of Compound **I**. The resulting solution is then treated with 11.11 mL, 20 mmol, of 1.8 M phenyllithium in dibutyl ether (Sigma-Aldrich) and after 15 minutes is cooled to -78°C with a dry ice bath. After a further 15 minutes, 3.92 g, 20 mmol, of Compound **S** dissolved in 5 mL of dry THF is added via syringe and the reaction mixture is warmed to -30°C through transfer of the apparatus to a cryostat. A second equivalent of 1.8 M phenyllithium is then added followed by excess methanol with the temperature maintained at -30°C. After stirring for 5 minutes the reaction is brought to room temperature and 40 mL of water is added and the reaction mixture is transferred to a 1 L separatory funnel where 200 mL of ethyl acetate is added. After being vigorously shaken, the biphasic mixture is separated and the aqueous phase is further extracted with two 50 mL portions of ethyl acetate. The combined organic phases are then dried with sodium sulfate and filtered through a plug of 1 inch of Celite via a 100 mL sinter funnel under vacuum into a 1 L RBF, with the sodium sulfate residue washed with a further 50 mL of ether. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give Compound **T.**

### Synthesis of Intermediate T (From Intermediates H and S)

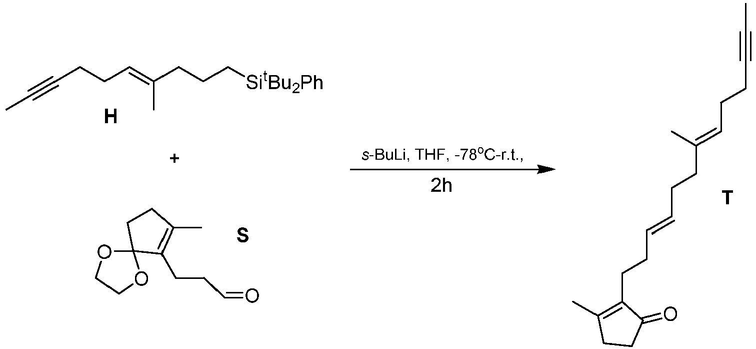

Compound T may be prepared by adapting the procedures of W. Adam, C. M. Ortega-Schulte, Synlett, 2003, 414-416 and A. Barbero, Y. Blanco, C. Garcia, Synthesis, 2000, 1223-1228. To a dried 250 mL RBF under an atmosphere of argon at room temperature is added 100 mL of distilled THF, and 9.82 g, 20 mmol, of Compound **H.** The resulting solution is then cooled to -78°C with a dry ice bath and 14.29 mL, 20 mmol, of 1.4 M sec-butyllithium in cyclohexane (Sigma-Aldrich) is added over 5 minutes. After a further 45 minutes, 3.92 g, 20 mmol, of Compound **S** dissolved in 5 mL of dry THF is added via syringe and the reaction mixture is warmed to room temperature After stirring for an additional 2 hours the reaction is diluted with 150 mL of ether and 40 mL of water is then added and the reaction mixture is transferred to a 1 L separatory funnel. After being vigorously shaken, the biphasic mixture is separated and the aqueous phase is further extracted with two 50 mL portions of ether. The combined organic phases are then dried with sodium sulfate and filtered through a plug of 1 inch of Celite via a 100 mL sinter funnel under vacuum into a 1 L RBF, with the sodium sulfate residue washed with a further 50 mL of ether. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give Compound **T.**

### Synthesis of Intermediate T (From Intermediates E and R)

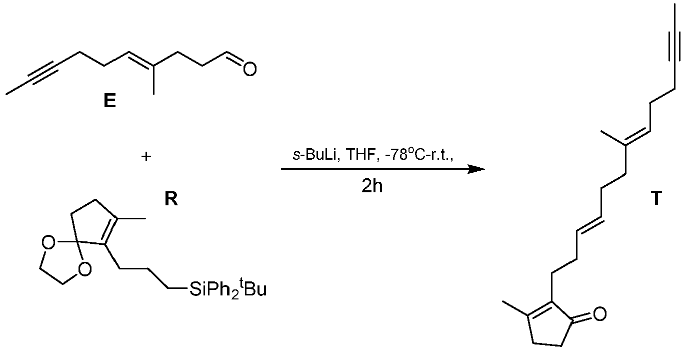

Compound T may be prepared by adapting the procedures of W. Adam, C. M. Ortega-Schulte, Synlett, 2003, 414-416 and A. Barbero, Y. Blanco, C. Garcia, Synthesis, 2000, 1223-1228, with different starting materials. To a dried 250 mL RBF under an atmosphere of argon at room temperature is added 100 mL of distilled THF, and 8.4 g, 20 mmol, of Compound **R.** The resulting solution is then cooled to - 78°C with a dry ice bath and 14.29 mL, 20 mmol, of 1.4 M sec-butyllithium in cyclohexane (Sigma-Aldrich) is added over 5 minutes. After a further 45 minutes, 2.68 g, 20 mmol, of Compound **E** dissolved in 5 mL of dry THF is added via syringe and the reaction mixture is warmed to room temperature After stirring for an additional 2 hours the reaction is diluted with 150 mL of ether and 40 mL of water is then added and the reaction mixture is transferred to a 1 L separatory funnel. After being vigorously shaken, the biphasic mixture is separated and the aqueous phase is further extracted with two 50 mL portions of ether. The combined organic phases are then dried with sodium sulfate and filtered through a plug of 1 inch of Celite via a 100 mL sinter funnel under vacuum into a 1 L RBF, with the sodium sulfate residue washed with a further 50 mL of ether. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give Compound **T.**

### Synthesis of Intermediate U

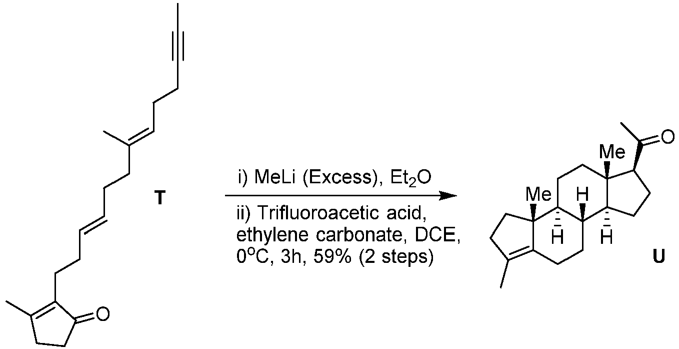

Compound **U,** may be prepared by adapting the procedures of Johnson, W. S.; Gravestock, M. B.; McCarry, B. E. J. Am. Chem. Soc., 1971, 93, 4332-4334. To a dried 250 mL RBF, equipped with a stirrer bar, under an atmosphere of argon at room temperature is added 100 mL of distilled ether and 5.68 g, 20 mmol, of Compound **T.** The resulting solution is then treated with 25 mL, 40 mmol, of 1.6 M methyllithium in ether (Sigma-Aldrich) at room temperature and the reaction mixture monitored by TLC. Once all the starting material is consumed the reaction is quenched with 25 mL of aqueous saturated ammonium chloride and transferred to a 1 L separatory funnel where an additional 200 mL of ether is added. After being vigorously shaken, the biphasic mixture is separated and the aqueous phase is further extracted with two 50 mL portions of ether. The combined organic phases are then dried with sodium sulfate and filtered through a plug of 1 inch of Celite via a 100 mL sinter funnel under vacuum into a 1 L RBF, with the sodium sulfate residue washed with a further 50 mL of ether. The collected solution is then reduced under vacuum on a Buchi rotary evaporator and the crude alcohol used without further purification due to instability. Thus in a 500 mL RBF, equipped with a stirrer bar, under argon, the crude alcohol is taken up in 200 mL of distilled dichloroethane (DCE), to which is added 59.5 g of ethylene carbonate. This mixture is then cooled to 0°C with an ice bath and 37 mL of trifluoroacetic acid is added via syringe. The reaction mixture is stirred for 3 hours before excess potassium carbonate in aqueous methanol (50 mL) is added and the reaction mixture is transferred to a 1 L separatory funnel. After being vigorously shaken, the biphasic mixture is separated and the aqueous phase is further extracted with two 50 mL portions of ether. The combined organic phases are then dried with sodium sulfate and filtered through a plug of 1 inch of Celite via a 100 mL sinter funnel under vacuum into a 1 L RBF, with the sodium sulfate residue washed with a further 50 mL of ether. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give Compound **U** as a racemic mixture.

### Synthesis of ent-progesterone (From Intermediate U)

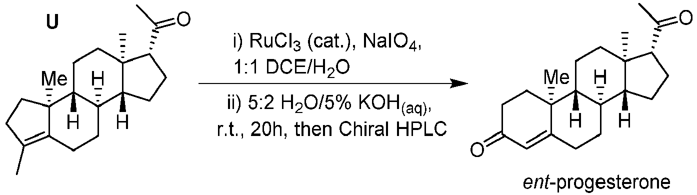

Step I may be prepared by adapting the procedures of Yang, D.; Zhang, C. J. Org. Chem., 2001, 66, 4814-4818; Step ii may be prepared by adapting the procedures of Johnson, W. S.; Gravestock, M. B.; McCarry, B. E. J. Am. Chem. Soc., 1971, 93, 4332-4334. To a 250 mL RBF, equipped with a stirrer bar, under an atmosphere of argon at room temperature is added 100 mL of a 1:1 mixture of DCE/H₂O, 5.68 g, 20 mmol, of Compound **U,** 0.145 g, 0.7 mmol of ruthenium(III)chloride (Sigma-Aldrich) and 8.56 g, 40 mmol of sodium periodate (Sigma-Aldrich). The reaction mixture is stirred at room temperature and monitored by TLC. Upon completion the reaction mixture is diluted with 100 mL of ether and transferred to a 500 mL separatory funnel where an additional. After being vigorously shaken, the biphasic mixture is separated and the aqueous phase is further extracted with two 50 mL portions of ether. The combined organic phases are then dried with sodium sulfate and filtered through a plug of 1 inch of Celite above 1 inch of flash silica (silica gel 60, EMD) via a 100 mL sinter funnel under vacuum into a 1 L RBF, with the sodium sulfate residue washed with a further 50 mL of ether. The collected solution is then reduced under vacuum on a Buchi rotary evaporator and the crude triketone used without further purification. Thus in a 100 mL RBF, equipped with a stirrer bar, under argon, the crude triketone is treated with 50 mL of 5:2 water/5% potassium hydroxide solution for 20 hours at room temperature. After which time 100 mL of ethyl acetate is added to the reaction mixture, which is then transferred to a 1 L separatory funnel. After being vigorously shaken, the biphasic mixture is separated and the aqueous phase is further extracted with two 50 mL portions of ethyl acetate. The combined organic phases are then dried with sodium sulfate and filtered through a plug of 1 inch of Celite via a 100 mL sinter funnel under vacuum into a 1 L RBF, with the sodium sulfate residue washed with a further 50 mL of ethyl acetate. The collected solution is then reduced under vacuum on a Buchi rotary evaporator. The residue is then taken up in a 5:1 mixture of hexane/ethyl acetate and purified by flash column chromatography (Silica gel 60, EMD, hexane/ethyl acetate solvent system) to give rac-progesterone. The enantiomers are subsequently separated with chiral HPLC to give *ent*-progesterone.

### Reference List

1. US Publication No. US 2005/0187188 to Stein et al., published August 25, 2005- "Methods for the Treatment of a Traumatic Central Nervous System Injury"
2. US Publication No. US 2007/0078117 to Hoffman et al., published April 5, 2007- "Methods for the Treatment of a Traumatic Central Nervous System Injury"
3. US Publication No. US 2008/0318914 to Hoffman et al., published December 25, 2008- "Methods for the Treatment of a Traumatic Central Nervous System Injury"
4. US Publication No. US 2009/0221544 to Stein et al., published September 3, 2009- "Methods for the Treatment of a Traumatic Central Nervous System Injury via a Tapered Administration Protocol"
5. US Publication No. US 2009/0325920 to Hoffman et al., published December 31, 2009- "Methods for the Treatment of a Traumatic Central Nervous System Injury"
6. US Publication No. US 2011/0306579 to Stein et al., published December 15, 2011- "Methods of Neuroprotection using Neuroprotective Steroids and a Vitamin D"
7. R. J. Auchus et al. "The Enantiomer of Progesterone (ent-progesterone) is a Competitive Inhibitor of Human Cytochromees P450c17 and P450c21," Archives of Biochemistry and Biophysics 409 (2003) 134-144
8. S. D. Rychnovsky et al. "Synthesis of ent-Cholesterol, the Unnatural Enantiomer," J. Org. Chem. 57 (1992) 2732-2736
9. S. Talengaonkar et al. "Intranasal Delivery: An Approach to Bypass the Blood Brain Barrier," Indian J. Pharmacol, Vol 36, Issue 3, (2004), 140-147
10. H. Nemoto et al. " First Enantioselective Total Synthesis of (+)-Cortisone," J. Org. Chem. 55 (1990) 5625-5631
11. W. S. Johnson et al. "Synthesis of dl-Progesterone," Journal of the American Chemical Society, Vol 93, Issue 17, (1971) 4332-4334
12. M. Weimar et al. "Enantioselective Synthesis of (+)-Estrone Exploiting a Hydrogen Bond-Promoted Diels-Alder Reaction," J. Org. Chem 75 (2010) 2718-2721
13. Herrmann et al. "Formal Total Synthesis of (±)-Estrone and Zirconocene-Promoted Cyclization of 2-Fluoro-1-7-octadienes and Ru-Catalyzed Ring Closing Metathesis," J. Org. Chem 73 (2008) 6202-6206
14. Q. Hu et al. "Simple, Catalytic Enantioselective Synthesis of Estrone and Desogestrel," J. Am Chem Soc 126 (2004) 5984-5986
15. Y. Horiguchi et al. "Total Synthesis of (±)-Cortisone. Double Hydroxylation Reaction for the Construction of Corticoid Side Chain," J. Org. Chem. 51 (1986) 4323-4325
16. US Patent Application No. 13/645,854 to VanLandingham et al. "Prophylactic and Post-Acute Use of Progesterone to Better Outcomes Associates with Concussion"
17. US Patent Application No. 13/645,881 to VanLandingham et al. "Nasal Delivery Mechanism for Prophylactic and Post-Acute Use for Progesterone and/or Its Enantiomer for Use in Treatment of Mild Traumatic Brain Injuries"
18. US Patent Application No. 13/645,925 to VanLandingham et al. "Prophylactic and Post-Acute Use of Progesterone in Conjunction with its Enantiomer for Use in Treatment of Mild Traumatic Brain Injuries"
19. International PCT Patent Application No. PCT/US2012/59030 to VanLandingham et al. "Prophylactic and Post-Acute Use of Progesterone to Better Outcomes Associated with Concussion"
20. International PCT Patent Application No. PCT/US2012/59087 to VanLandingham et al. "Nasal Delivery Mechanism for Prophylactic and Post-Acute Use for Progesterone and/or Its Enantiomer for Use in Treatment of Mild Traumatic Brain Injuries"
21. International PCT Patent Application No. PCT/US2012/59083 to VanLandingham et al. "Prophylactic and Post-Acute Use of Progesterone in Conjunction with its Enantiomer for Use in Treatment of Mild Traumatic Brain Injuries"
22. Anand Kumar, T.C., David, G.F.X., Sankaranarayanan, A., Puri, V. and Sundram, K. R., 1982. Pharmacokinetics of progesterone after its administration to ovariectomized rhesus monkeys by injection, infusion or intranasal spraying. Proceedings of the National Academy of Science 79, 4185-4189.
23. Anand Kumar, T.C., David, G.F.X., Kumar, K., Umberkoman, B. and Krishnamoorthy, M.S., 1974b. A new approch to fertility regulation by interfering with neuroendochrine pathways. In: Neuroendocrine Regulation of Fertility. Karger, Basel, 314-322.
24. Anand Kumar, T.C., David, G.F., Umberkoman, B. and Saini, K.D., 1974a. Uptake of radioactivity by body fluids and tissues in rhesus monkeys after intravenous injection or intranasal spray of tritium-labelled oestradiol and progesterone. Curr. Sci. 43, 435439.
25. Cincinelli et al., "Effects of the repetitive administration of progesterone by nasal spray in postmeonpausal women," Fertil. Steril. 60(6): 1020-1024 (1993).
26. Cincinelli et al., "Nasal spray administration of unmodified progesterone: evaluation of progesterone serum levels with three different radioimmunoassay techniques," Maturitas J. of the Climacteric & Postmenopause 19(1): 43-52 (1994).
27. Cincinelli et al., "Nasally-administered progesterone: comparison of ointment and spray formulations," Maturitas 13(4): 313-317 (1991).
28. Cincinelli et al., "Progesterone administration by nasal spray in menopausal women: comparison between two different spray formulations," Gynecol. Endocrinol. 6(4): 247-251 (1992).
29. Cincinelli et al., "Progesterone administration by nasal spray," Fertil. & Steril. 56(1): 139-141 (July 1991).
30. Provasi et al., " Nasal Delivery Progesterone Powder Formulations Comparison with Oral Administration," Boll. Chim. Farm. 132(10): 402-404 (1993).
31. van Wingen GA, van Broekhoven F, Verkes RJ, Petersson KM, Backstrom T, Buitelaar JK, Fernandez G. Progesterone selectively increases amygdala reactivity in women. Mol Psychiatry. 2007:in press.
32. Buitelaar JK, et al., Progesterone selectively increases amygdala reactivity in women. Mol Psychiatry: 13,325-333 (2008); doi:10.1038/sj.mp.4002030; published online 19 June 2007.
33. Steege et al., "Bioavailability of nasally administered progesterone," Fertil. Steril. 46(4): 727-729 (1986).
34. Grace Rathnam, N. Narayanan, and R. Ilavarasan, Carbopol-Based Gels for Nasal Delivery of Progesterone. AAPS PharmSciTech. 2008 December; 9(4): 1078-1082.
35. S.K. Jain, A. Jain, Y. Gupta, U. Gupta: Progesterone bearing mucoadhesive carriers for nasal delivery. J. Drug Del. Sci. Tech., 17 (2) 137-143 2007.
36. Diane C. Corbo, Yih C. Huang, Yie W. Chien: Nasal delivery of progestational steroids in ovariectomized rabbits: I. progesterone - comparison of pharmacokinetics with intravenous and oral administration, International Journal of Pharmaceutics, Volume 46, Issues 1-2, September 1988, Pages 133-140.
37. Bioadhesive Drug Delivery Systems: Fundamentals, Novel Approaches. Edith Mathiowitz, Donald E. Chickering, Claus-Michael Lehr - 1999 - 670 pages, (1996) in which the effect of particle size of nasal powders of [3-cyclodextrin containing progesterone on delivery behavior (including bioavailability) was studied). See Page 525.
38. Brittebo & Rafter, "Steroid Metabolism by Rat Nasal Mucosa: Studies on Progesterone and Testosterone," J. Steroid Biochem. 20(5): 1147-1151 (1984).

### Incorporation By Reference

The entire contents of all patents published patent applications and other references cited herein are hereby expressly incorporated herein in their entireties by reference.

### Equivalents

Those skilled in the art will recognize, or be able to ascertain using no more than routine experimentation, numerous equivalents to the specific procedures described herein. Such equivalents are considered to be within the scope of this invention and are covered by the following claims.

## Claims

1. A method for preparing ent-progesterone comprising the step of reacting a compound of the formula: 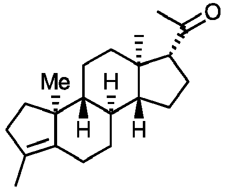 with a ruthenium catalyst and an oxidizing agent to prepare ent-progesterone.

2. A method for preparing ent-progesterone according to claim 1 further comprising the step of reacting a compound of formula A: 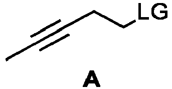 with metal bromide to produce 5-bromopent-2-yne, wherein LG represents a leaving group.

3. A method for preparing ent-progesterone according to claim 1 further comprising the step of reacting a compound of formula D: 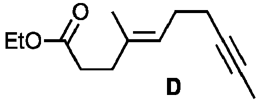 with diisobutylaluminum hydride to form a compound of formula E:

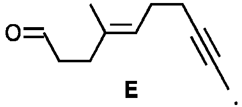

4. A method for preparing ent-progesterone according to claim 1 further comprising the step of reacting a compound of formula K: 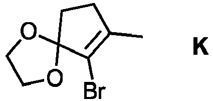 with a compound of formula M: 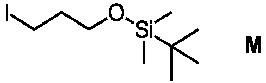 to form a compound of formula N:

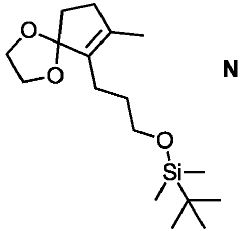

5. A method for preparing ent-progesterone according to claim 1 further comprising the step of reacting a compound of formula H: 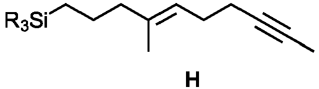 with a compound of formula S: 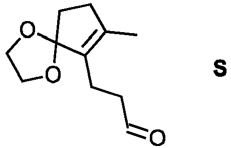 to form a compound of formula T: 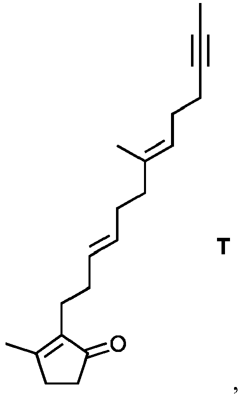 wherein each instance of R is independently a C1-C4 straight or branched alkyl group, or a C3-C8 cycloalkyl group.

6. A method for preparing ent-progesterone according to claim 1 further comprising the step of reacting a compound of formula E: 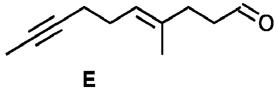 with a compound of formula R: 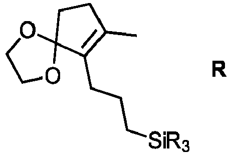 to form a compound of formula T: 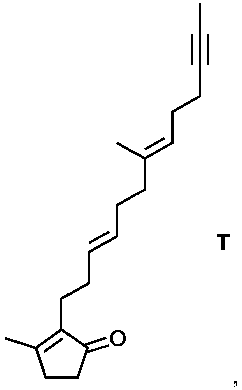 wherein each instance of R is independently a C1-C4 straight or branched alkyl group, or a C3-C8 cycloalkyl group.

7. A method for preparing ent-progesterone according to claim 1 further comprising the step of reacting a compound of formula E: 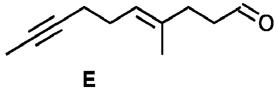 with a compound of formula Q: 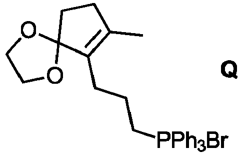 to form a compound of formula T:

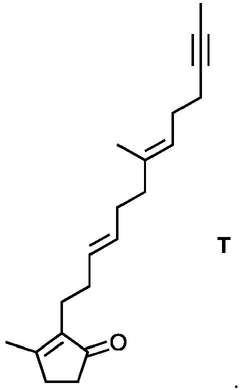

8. A method for preparing ent-progesterone according to claim 1 further comprising the step of reacting a compound of formula I: 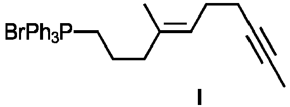 with a compound of formula S: 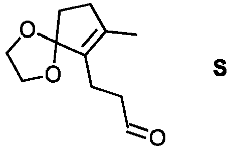 to form a compound of formula T:

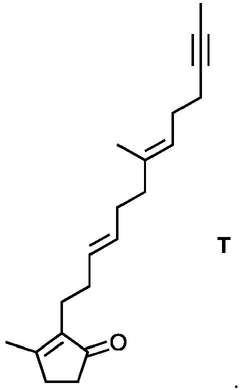

9. A method for preparing the trienone precursor to ent-progesterone comprising the step of reacting a compound of formula: 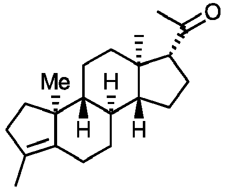 with a ruthenium catalyst and an oxidizing agent to prepare ent-progesterone.

## Patentansprüche

1. Verfahren zum Zubereiten von Ent-Progesteron, mit dem Schritt von Reagieren einer Verbindung der Formel: 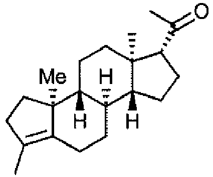 mit einem Ruthenium-Katalysator und einem Oxidationsmittel zum Zubereiten von Ent-Progesteron.

2. Verfahren zum Zubereiten von Ent-Progesteron gemäß Anspruch 1, ferner mit dem Schritt von Reagieren einer Verbindung von Formel A: 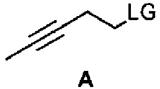 mit einem Metallbromid zum Herstellen von 5-Bromopent-2-in, bei dem LG eine Abgangsgruppe darstellt.

3. Verfahren zum Zubereiten von Ent-Progesteron gemäß Anspruch 1, ferner mit dem Schritt von Reagieren einer Verbindung von Formel D: 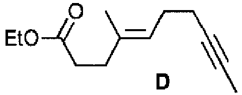 mit Diisobutylaluminiumhydrid zum Ausbilden einer Verbindung von Formel E:

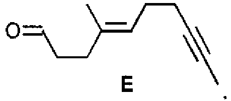

4. Verfahren zum Zubereiten von Ent-Progesteron gemäß Anspruch 1, ferner mit dem Schritt von Reagieren einer Verbindung von Formel K: 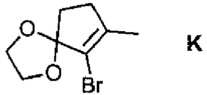 mit einer Verbindung von Formel M: 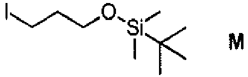 zum Ausbilden einer Verbindung von Formel N:

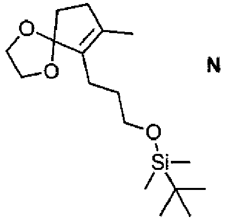

5. Verfahren zum Zubereiten von Ent-Progesteron gemäß Anspruch 1, ferner mit dem Schritt von Reagieren einer Verbindung von Formel H: 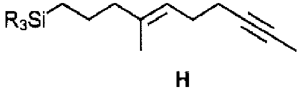 mit einer Verbindung von Formel S: 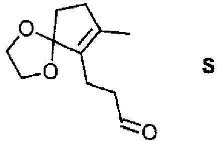 zum Ausbilden einer Verbindung von Formel T: 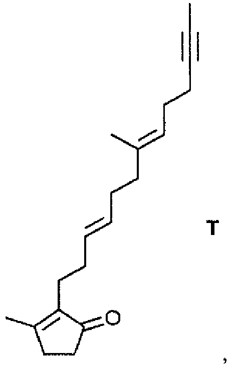 bei dem jede Instanz von R unabhängig eine gerade oder verzweigte C1-C4-Alkylgruppe oder eine C3-C8-Cycloalkylgruppe ist.

6. Verfahren zum Zubereiten von Ent-Progesteron gemäß Anspruch 1, ferner mit dem Schritt von Reagieren einer Verbindung von Formel E: 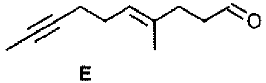 mit einer Verbindung von Formel R: 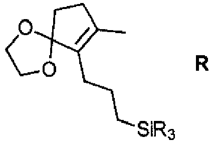 zum Ausbilden einer Verbindung von Formel T: 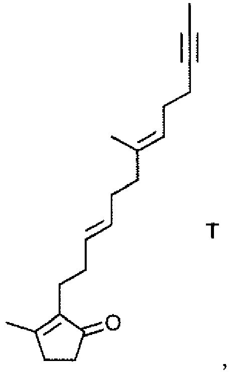 bei dem jede Instanz von R unabhängig eine gerade oder verzweigte C1-C4-Alkylgruppe oder eine C3-C8-Cycloalkylgruppe ist.

7. Verfahren zum Zubereiten von Ent-Progesteron gemäß Anspruch 1, ferner mit dem Schritt von Reagieren einer Verbindung von Formel E: 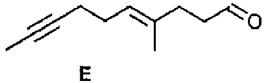 mit einer Verbindung von Formel Q: 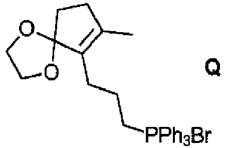 zum Ausbilden einer Verbindung von Formel T:

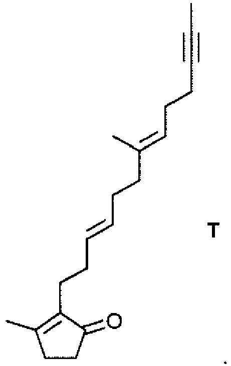

8. Verfahren zum Zubereiten von Ent-Progesteron gemäß Anspruch 1, ferner mit dem Schritt von Reagieren einer Verbindung von Formel I: 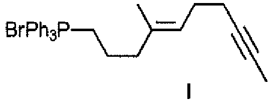 mit einer Verbindung von Formel S: 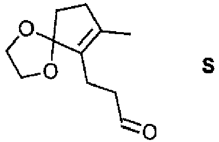 zum Ausbilden einer Verbindung von Formel T:

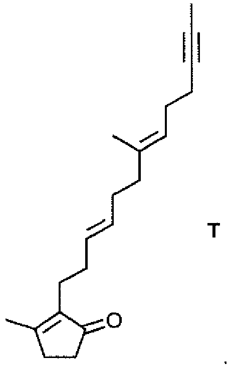

9. Verfahren zum Zubereiten des Trienon-Vorgängers zu Ent-Progesteron, mit dem Schritt von Reagieren einer Verbindung von Formel: 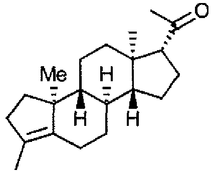 mit einem Ruthenium-Katalysator und einem Oxidationsmittel zum Zubereiten von Ent-Progesteron.

## Revendications

1. Procédé pour préparer de l'ent-progestérone, comprenant l'étape de réaction d'un composé de formule : 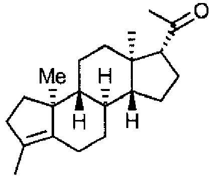 avec un catalyseur au ruthénium et un agent d'oxydation, pour préparer de l'ent-progestérone.

2. Procédé pour préparer de l'ent-progestérone selon la revendication 1, comprenant en outre l'étape de réaction d'un composé de formule A : 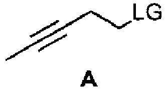 avec un bromure métallique pour produire du 5-bromopent-2-yne, formule dans laquelle LG représente un groupe partant.

3. Procédé pour préparer de l'ent-progestérone selon la revendication 1, comprenant en outre l'étape de réaction d'un composé de formule D : 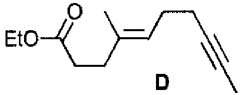 avec de l'hydrure de diisobutyl-aluminium, pour former un composé de formule E :

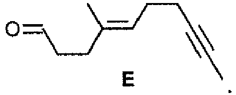

4. Procédé pour préparer de l'ent-progestérone selon la revendication 1, comprenant en outre l'étape de réaction d'un composé de formule K : 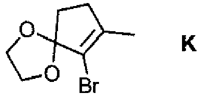 avec un composé de formule M : 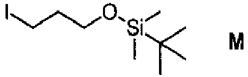 pour former un composé de formule N :

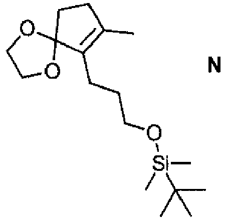

5. Procédé pour préparer de l'ent-progestérone selon la revendication 1, comprenant en outre l'étape de réaction d'un composé de formule H : 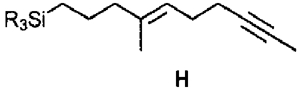 avec un composé de formule S : 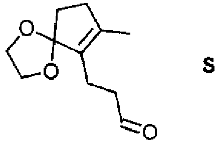 pour former un composé de formule T : 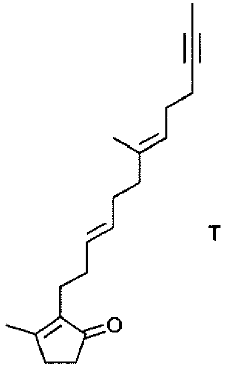 où, dans chaque cas, R est indépendamment un groupe alkyle linéaire ou ramifié en C₁ à C₄, ou un groupe cycloalkyle en C₃ à C₈.

6. Procédé pour préparer de l'ent-progestérone selon la revendication 1, comprenant en outre l'étape de réaction d'un composé de formule E : 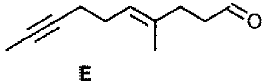 avec un composé de formule R : 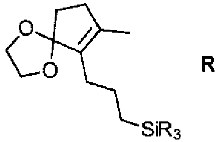 pour former un composé de formule T : 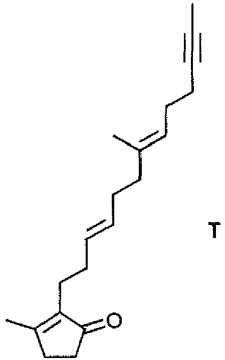 où, dans chaque cas, R est indépendamment un groupe alkyle linéaire ou ramifié en C₁ à C₄, ou un groupe cycloalkyle en C₃ à C₈.

7. Procédé pour préparer de l'ent-progestérone selon la revendication 1, comprenant en outre l'étape de réaction d'un composé de formule E : 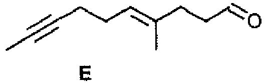 avec un composé de formule Q : 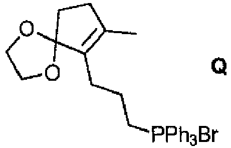 pour former un composé de formule T :

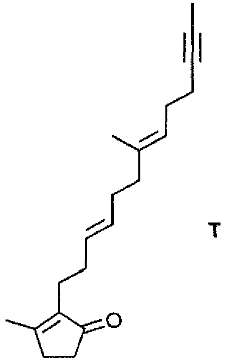

8. Procédé pour préparer de l'ent-progestérone selon la revendication 1, comprenant en outre l'étape de réaction d'un composé de formule I : 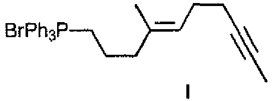 avec un composé de formule S : 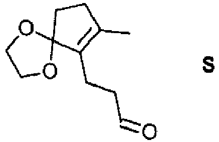 pour former un composé de formule T :

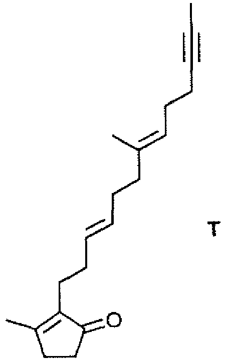

9. Procédé pour préparer le précurseur triénone en ent-progestérone, comprenant l'étape de réaction d'un composé de formule : 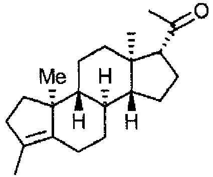 avec un catalyseur au ruthénium et un agent d'oxydation, pour préparer de l'ent-progestérone.
